# EUROPÄISCHE PATENTSCHRIFT

(11) **EP 3 382 414 B1**
(45) Veröffentlichungstag und Bekanntmachung des Hinweises auf die Patenterteilung: **18.09.2024**
(21) Anmeldenummer: 17163305.0
(22) Anmeldetag: 28.03.2017
(51) Int. Cl.: G01R 33/54, G01R 33/563

(54) **VERFAHREN ZUR DURCHFÜHRUNG EINER ANGIOGRAPHISCHEN MESSUNG UND ERSTELLUNG EINER ANGIOGRAPHIE**
METHOD FOR PERFORMING AN ANGIOGRAPHIC MEASUREMENT AND GENERATION OF AN ANGIOGRAPHY
PROCÉDÉ D'EXÉCUTION D'UNE MESURE ANGIOGRAPHIQUE ET D'ÉTABLISSEMENT D'UNE ANGIOGRAPHIE

(43) Veröffentlichungstag der Anmeldung: 03.10.2018
(73) Patentinhaber: Siemens Healthineers AG, 91301 Forchheim (DE)
(72) Erfinder: KEIL, Miriam, 91056 Erlangen-Dechsendorf (DE); KESSNER, Martin, 80336 München (DE)
(74) Vertreter: Siemens Healthineers Patent Attorneys

(56) Entgegenhaltungen:
- DE-A1- 10 230 877
- DE-A1- 102007 009 185
- US-A- 5 928 148
- US-A1- 2003 095 150

## Beschreibung

Die Erfindung betrifft ein Verfahren zur Durchführung einer angiographischen Messung und Erstellung einer Angiographie von einem Körperbereich eines Patienten in einer Magnetresonanzanlage, wobei der zu untersuchende Körperbereich größer als ein maximales Gesichtsfeld der Magnetresonanzanlage ist und die Magnetresonanzanlage eine Steuereinheit zur Steuerung der Durchführung der angiographischen Messung und der Erstellung der Angiographie aufweist, sowie die zugehörige Magnetresonanzanlage.

In einer Magnetresonanzanlage wird üblicherweise der zu untersuchende Körper einer Untersuchungsperson, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, insbesondere Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Die kontrastverstärkte Magnetresonanztomographie ist eine bekannte Untersuchungsmethode zur Darstellung des arteriellen und venösen Gefäßsystems. Die Durchführung einer kontrastverstärkten Magnetresonanztomographie der Extremitäten gehört zu den anspruchsvolleren Aufgaben der medizinischen Bildgebung.

Da die heute auf dem Markt erhältlichen Magnetresonanzanlagen üblicherweise ein Field-of-View von ungefähr 50cm in Längsrichtung aufweisen, muss der Patient während der Untersuchung innerhalb der Magnetresonanzanlage bewegt werden, um den Patienten beispielsweise von den Füßen bis zum Thorax zu erfassen. Eine derartige Messung wird daher in Teilangiographiemessungen unterteilt und separat durchgeführt. Das Festlegen der Sequenzparameter und des zeitlichen Ablaufs der Teilangiographiemessungen erfordert besondere Aufmerksamkeit, insbesondere wenn Kontrastmittel (auch als KM bezeichnet) injiziert wird, welches das Signal der Blutgefäße gegenüber dem Signal des umgebenden Gewebes verstärkt, und dessen Verteilung im Körper erfasst werden soll, wie dies bei der angiographischen Messung der Fall ist. Durch Datenakquisition vor und nach Kontrastmittelinjektion kann mittels der Subtraktion das umgebende Gewebe fast vollständig eliminiert werden.

Aus DE 10 2013 220 288 B4 ist ein Verfahren zum Aufnehmen von Magnetresonanz-Bilddaten, eine Bilddatenaufnahmeeinheit, ein Magnetresonanzgerät und ein Computerprogrammprodukt bekannt, bei welchem Steuerbefehle für eine Magnetresonanzanlage optimiert und überprüft werden.

Die DE 10 2009 031 164 B4 offenbart ein Verfahren zur Durchführung einer Multistep-Messung in einer Magnetresonanz-Anlage im Zusammenhang mit einer Angiographie.

Die DE 10 2011 007 835 A1 beschreibt ein Verfahren zur kontrastmittelfreien Magnetresonanz-Angiographie, welche eine vergleichsweise aufwendige Planung erfordert, und in der DE 10 2006 006 309 B4 ist ein Verfahren offenbart, bei welchem während der Aufzeichnung von Magnetresonanz-Daten eine Tischverschiebung durchgeführt wird.

Die US 2003/0095150 A1 offenbart ein Verfahren zur einfacheren Planung einer peripher-vaskulären Untersuchung (PV) mittels einer Magnetresonanz-Anlage.

Aus der DE 10 2007 009 185 A1 ist ein Verfahren zur Planung einer angiographischen Messung von einem Körperbereich in einer Magnetresonanzanlage, der größer als das maximale Gesichtsfeld der Magnetresonanzanlage ist, bekannt.

Der Erfindung liegt die Aufgabe zu Grunde, ein Verfahren bereitzustellen, das es ermöglicht, eine angiographische Messung mit geringer und komfortabel ausgestalteter Interaktion mit einem Benutzer, durchzuführen, und gleichzeitig die Patientenvariabilität adäquat berücksichtigt. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zur Durchführung einer angiographischen Messung und Erstellung einer Angiographie von einem Körperbereich eines Patienten in einer Magnetresonanzanlage, wobei der Körperbereich größer als ein maximales Gesichtsfeld der Magnetresonanzanlage ist und die Magnetresonanzanlage eine Steuereinheit zur Steuerung der Durchführung der angiographischen Messung und der Erstellung der Angiographie aufweist, weist folgende Schritte auf:
- Erfassen einer anatomischen Größe des Patienten und einer oberen Grenze und einer unteren Grenze des Körperbereichs,
- Aufteilen der angiographischen Messung in Teilangiographiemessungen, wobei die Steuereinheit den Körperbereich in mehrere, nacheinander zu messende Teilmessbereiche aufteilt, wobei jeder Teilmessbereich unterschiedlichen Körperregionen zugeordnet ist, wobei jeder Teilmessbereich nicht größer als das maximale Gesichtsfeld ist und je einer Teilangiographiemessung zugeordnet ist, und wobei jeder Teilangiographiemessung Messzeitpunkte, umfassend jeweils eine Messstartzeit und eine Messendzeit, zugeordnet sind, so dass eine Gesamtmessdauer der angiographischen Messung durch die Messstartzeit der ersten Teilangiographiemessung und die Messendzeit der letzten Teilangiographiemessung festgelegt ist,
- Anzeigen der Messstartzeiten, der Messzeitdauer und der Messendzeiten der Teilangiographiemessungen auf einem graphischen Userinterface,
- Verändern der Messzeitpunkte, wobei bei der Veränderung eines Messzeitpunkts einer Teilangiographiemessung automatisch eine Anpassung mindestens eines weiteren Messzeitpunkts einer anderen Teilangiographiemessung erfolgt,
- Automatisches Festlegen von Sequenzparametern der Teilangiographiemessungen basierend auf den veränderten Messstartzeiten und/oder Messendzeiten derart, dass die Teilangiographiemessung zwischen zugehöriger Messstartzeit und Messendzeit durchführbar ist, so dass die Teilangiographiemessung innerhalb der vorgegebenen Messzeitdauer abgeschlossen sein muss,
- Bereitstellen der Sequenzparameter der Steuereinheit der Magnetresonanzanlage,
- Durchführen der Teilangiographiemessungen, und
- Erstellen der Angiographie anhand der durchgeführten Teilangiographiemessungen.

Besondere Bedeutung kommt typischerweise neben dem Aufteilen des zu untersuchenden Körperbereichs in geometrische Teilmessbereiche dem Festlegen des zeitlichen Ablaufs der angiographischen Messung zu, um die Datenakquisition zeitlich exakt mit der Kontrastmittelinjektion zu synchronisieren, so dass der Kontrastmittelbolus während der Datenakquisition den Teilmessbereich passiert. Insbesondere müssen daher die zugeordneten Teilangiographiemessungen in ihren Sequenzparametern und insbesondere in ihren Messzeitpunkten wie Messzeitdauer, Messstartzeiten und Messendzeiten angepasst werden, um patientenspezifische Charakteristika wie die Zirkulationszeit des Kontrastmittels und die Patientengröße adäquat zu berücksichtigen. Um eine Überlagerung der Arterien durch die Venen zu verhindern, ist es zusätzlich erforderlich, dass die Durchführung der angiographischen Messung vor dem Anfluten des Kontrastmittels in den Venen (Rückfluss) beendet ist. Bisher erfolgte üblicherweise die Durchführung der angiographischen Messung mittels aufwändiger und ausgiebiger Interaktion mit dem Benutzer, welcher sowohl die Geometrie mittels der Teilmessbereiche der angiographischen Messung als auch den zeitlichen Ablauf der jeweils zugeordneten Teilangiographiemessungen der angiographischen Messung derart festlegen musste, dass die Teilmessbereiche der Teilangiographiemessungen im maximalen Gesichtsfeld der Magnetresonanzanlage liegen.

Zur Unterstützung des Vorgehens wird typischerweise eine Kontrastmitteldynamik im Abdomen erfasst und darauf basierend z.B. der Teilmessbereich Abdomen mit der entsprechenden Teilangiographiemessung angepasst. Entweder aus Zeitgründen oder aufgrund des fehlenden Fachwissens des Benutzers erfolgte üblicherweise keine Anpassung der anderen Teilmessbereiche an die Physiologie des Patienten (z.B. an eine Kontrastmitteldynamik, insbesondere an die Zirkulationszeit des Kontrastmittels). Insbesondere diese Anpassung der Messzeitpunkte auf die Kontrastmitteldynamik ist eine Aufgabe, die eine Vielzahl von Benutzerinteraktion-Schritten, welche in mehreren Teilschritten durchgeführt werden, abhängig insbesondere von der Anzahl der Teilangiographiemessungen erfordern kann. Durch das automatische Anpassen eines Messzeitpunkts, sobald ein anderer Messzeitpunkt verändert wird, kann die angiographische Messung schneller und komfortabler durchgeführt werden, weil weniger Benutzerinteraktion-Schritte zur Anpassung der Messzeitpunkte an beispielsweise die Teilmessbereiche, die Zirkulationszeit des Patienten und insbesondere der Kontrastmitteldynamik nötig sind.

Darüber hinaus werden die Sequenzparameter der Teilangiographiemessungen automatisch festgelegt, wodurch ebenfalls die angiographische Messung einfacher durchgeführt werden kann. Insbesondere die Entscheidung, welche der Vielzahl an Einstell- und Konfigurationsmöglichkeiten gewählt werden soll, konnte den Benutzer überfordern und ihn für eine längere Zeit in Beschlag nehmen. Dieser Zeitaufwand fiel bei jeder einzelnen Anpassung der Geometrie der Teilmessbereiche oder des zeitlichen Ablaufs der Teilangiographiemessungen an. Nun werden Sequenzparameter der Teilangiographiemessungen automatisch festgelegt, insbesondere bei jeder Veränderung des Messzeitpunkts und bei der damit verbundenen automatischen Anpassung des weiteren Messzeitpunkts. Hierdurch wird eine optimale und einfache Durchführung der angiographischen Messung möglich, insbesondere wenn der Benutzer über weniger stark ausgeprägtes Wissen über Sequenzparameter und sonstige Einstellungen bei der angiographischen Messung verfügt.

Eine anatomische Größe des Patienten kann insbesondere durch den Benutzer auf dem graphischen Userinterface oder alternativ automatisch mittels Bestimmung anhand von Landmarken erfasst werden. Die Körpergröße des Patienten von der Fußsohle bis zum Scheitel ist eine zweckmäßige anatomische Größe des Patienten. Üblicherweise wird die Körpergröße und das Körpergewicht bei Messungen in Magnetresonanzanlagen erfasst, um die spezifische Absorptionsrate des Patienten bestimmen zu können. Die Körpergröße und das Körpergewicht des Patienten können beispielsweise durch einen Benutzer manuell eingegeben werden, zum Beispiel unmittelbar im Rahmen der durchzuführenden angiographischen Messung, oder in einer weiterführenden Ausgestaltung automatisch aus einer Patientenakte übernommen werden, auf die die Magnetresonanzanlage Zugriff hat.

Ein Untersuchungsbereich stellt typischerweise ein Volumen, insbesondere den Körperbereich dar, welcher mittels der Magnetresonanzanlage abzubilden ist. Der Untersuchungsbereich wird typischerweise durch einen Benutzer, beispielsweise auf einer Übersichtsaufnahme (Localizer) durch Erfassen einer oberen Grenze und einer unteren Grenze des Körperbereichs festgelegt. In einer Ausgestaltung kann der Untersuchungsbereich alternativ mittels einer Auswahl der in einer Liste auf dem graphischen Userinterface aufgeführten Körperregionen festgelegt werden. Der Körper des Patienten kann zweckmäßigerweise gemäß seiner Anatomie in unterschiedliche Körperregionen wie Oberschenkel, Unterschenkel, Abdomen etc. aufgeteilt werden. In einer weiteren Ausgestaltung wird der Untersuchungsbereich auf dem graphischen Userinterface in einem Schema, das einem menschlichen Körper entspricht und typischerweise nach Position von Körperregionen aufgeteilt ist, festgelegt. Beispielsweise kann der Untersuchungsbereich auch automatisch aus der Terminplanung übernommen werden. Zweckmäßigerweise, wenn die Auswahl der Körperregionen in der Liste oder beispielsweise in dem Schema auf dem graphischen Userinterface erfolgt, wird dem Untersuchungsbereich als die obere Grenze und die untere Grenze des Körperbereichs entsprechend der ausgewählten Körperregionen entweder Standardpositionen oder Patientenpositionen anhand von Landmarken, die auf Übersichtsbildern, beispielsweise auf der Übersichtsaufnahme des Patienten erfasst wurden, automatisch zugeordnet.

Das maximale Gesichtsfeld der Magnetresonanzanlage, beispielsweise auch Field-of-View (FOV), insbesondere auch maximale Ausdehnung des FOV in z-Richtung der Magnetresonanzanlage genannt, umfasst ein Volumen, innerhalb dessen Messungen in einer Magnetresonanzanlage, insbesondere Teilangiographiemessungen, ohne Tischvorschub oder Umlagerung des Patienten durchgeführt werden können. Weil typischerweise der Körperbereich der angiographischen Messung größer als das maximale Gesichtsfeld der Magnetresonanzanlage ist, teilt die Steuereinheit den Körperbereich in mehrere Teilmessbereiche auf und ordnet die Teilmessbereiche jeweils im maximalen Gesichtsfeld der Magnetresonanzanlage an. Die Teilmessbereiche des Körperbereichs ergeben insbesondere zusammen den gesamten Untersuchungsbereich, der größer als das maximale Gesichtsfeld der Magnetresonanzanlage ist. Vorzugweise ordnet die Steuereinheit die Teilmessbereiche derart an, dass die Teilmessbereiche sich zweckmäßigerweise an den Rändern der Teilmessbereiche teilweise überlappen. Die Steuereinheit teilt den Körperbereich in mehrere Teilmessbereiche bevorzugt automatisch auf, wobei das Aufteilen beispielsweise auch mittels des Benutzers auf dem graphischen Userinterface durchgeführt werden kann. Des Weiteren kann ein Teilmessbereich beispielsweise bzgl. des umfassenden Volumens abgeändert werden.

Des Weiteren wird die angiographische Messung vorzugweise automatisch in Teilangiographiemessungen aufgeteilt, falls der von der oberen Grenze und der unteren Grenze des Körperbereichs definierte Bereich größer ist als das maximale Gesichtsfeld der Magnetresonanzanlage. Üblicherweise wird jedem Teilmessbereich, der nach der vorherigen Ausgestaltung gemäß der Aufteilung des Körperbereichs festgelegt worden ist, eine Teilangiographiemessung zugeordnet.

Jeder Teilangiographiemessung sind Messzeitpunkte, zumindest eine Messstartzeit als Messstart der Teilangiographiemessung und eine Messendzeit als Messende der Teilangiographiemessung, zugeordnet, wobei die Differenz zwischen Messendzeit und Messstartzeit der Messzeitdauer der Teilangiographiemessung entspricht. Jede einem unterschiedlichen Teilmessbereich zugeordnete Teilangiographiemessung unterscheidet sich von den anderen Teilangiographiemessungen typischerweise bei den Messzeitpunkten, insbesondere in der Messstartzeit und in der Messendzeit, weil die Teilangiographiemessungen oft nur nacheinander durchgeführt werden können. Die Teilangiographiemessung mit der geringsten Messtartzeit entspricht vorzugweise der ersten Teilangiographiemessung und die Teilangiographiemessung mit der höchsten Messstartzeit der letzten Teilangiographiemessung. Die Gesamtmessdauer der angiographischen Messung ist durch die Messstartzeit der ersten Teilangiographiemessung und die Messendzeit der letzten Teilangiographiemessung festgelegt. Zwischen den Teilangiographiemessungen sind üblicherweise Messzeitpausen angeordnet. Die Gesamtmessdauer setzt sich aus der Summe der Messzeitdauer der Teilangiographiemessungen sowie den Messzeitpausen zusammen. Eine Messzeitpause ist charakterisiert durch den Abstand zwischen der Messendzeit der vorherigen Teilangiographiemessung und der Messstartzeit der nachfolgenden Teilangiographiemessung und wird vorzugweise so kurz wie möglich gehalten. Die Länge der Messzeitpause ist insbesondere von der technischen Ausführung der Magnetresonanzanlage, beispielsweise dem maximalen Gesichtsfeld und der Geschwindigkeit des Tischvorschubs abhängig. Die Länge der Messzeitpause kann außerdem von der Dauer, die zum Ausspielen von akustischen Atemkommandos zum Atemanhalten oder zum Weiteratmen nötig ist, oder von Justagemaßnahmen für nachfolgende Teilangiographiemessungen abhängig sein. Beispielsweise kann die Teilangiographiemessung in dem zugeordneten Teilmessbereich Abdomen Atemkommandos zum Atemanhalten oder zum Weiteratmen erfordern während die Teilangiographiemessung in dem zugeordneten Teilmessbereich Füße keine Atemkommandos erfordert. In diesem Fall kann die Messzeitpause vor der Teilangiographiemessung in dem Teilmessbereich Füße kürzer ausfallen als wenn das Atemkommando zum Atemanhalten erforderlich wäre. Während der Messzeitpause wird der Patient beispielsweise durch Tischvorschub derart umgeleitet, dass der Teilmessbereich, der der darauffolgenden Teilangiographiemessung zugeordnet ist, im Gesichtsfeld der Anlage positioniert wird. Alternativ kann eine Umlagerung des Patienten erfolgen. Je Gesamtmessdauer gibt es üblicherweise bei einer Anzahl N an Teilangiographiemessungen *(N-1)* Messzeitpausen, je eine zwischen zwei Teilangiographiemessungen.

Jede Teilangiographiemessung umfasst üblicherweise genau eine Messung in dem zugeordneten Teilmessbereich in einer Magnetresonanzanlage. Die Teilangiographiemessungen sind vorzugs-weise derart zeitlich sequentiell angeordnet, dass die Reihenfolge der Teilangiographiemessungen dem voraussichtlichen Verlauf des Kontrastmittels im Patienten folgt. Zweckmäßigerweise ist die Messzeitdauer der Teilangiographiemessungen ebenso auf die Kontrastmitteldynamik des Patienten abgestimmt. Insbesondere mittels geeigneter Algorithmen kann nach Durchführung der Teilangiographiemessungen die Angiographie des Patienten ermittelt werden. Besonders vorteilhaft ist die automatische Aufteilung in Teilangiographiemessungen, wobei die Durchführung der Teilangiographiemessungen vereinfacht wird.

Typischerweise werden die Messstartzeiten, die Messzeitdauer, die Messendzeiten der Teilangiographiemessungen, die Gesamtmessdauer und eine Bolusverzögerung auf einem graphischen Userinterface vorzugweise auf einem Zeitstrahl angezeigt. Das Anzeigen kann beispielsweise einen Monitor umfassen. Der Zeitstrahl ist vorzugweise als horizontale Achse ausgebildet. Die Bolusverzögerung entspricht üblicherweise der Differenz zwischen Bolus-Injektion und der Messstartzeit der ersten Teilangiographiemessung, wobei nicht jede Teilangiographiemessung eine Bolus-Injektion erfordert. Beispielsweise werden daher weniger oder auch zusätzliche für die Durchführung der angiographischen Messung relevante Parameter angezeigt. Darüber hinaus kann je eine Messbox für eine Teilangiographiemessung über dem Zeitstrahl, deren Breite durch die Messstartzeit und die Messendzeit der Teilangiographiemessung definiert ist, angezeigt werden.

Üblicherweise können die Messzeitpunkte der Teilangiographiemessungen verändert werden. Die Messzeitpunkte können vorzugweise durch Interaktionsschritte des Benutzers mit dem graphischen Userinterface verändert werden. Das Verändern kann das Verschieben entlang des Zeitstrahls mittels Auswahl eines Messpunkts mit einem Eingabegerät und einem nachfolgenden Freigeben des Messpunkts bedeuten, üblicherweise auch "Drag and Drop" genannt. Alternativ kann auch mittels Eingabe von einem Zahlenwert oder Auswahl automatisch vorgegebener Werte durch den Benutzer eine Verschiebung eines Messzeitpunkts entlang des Zeitstrahls bestimmt werden. Andere Formen der Interaktion des Benutzers auf oder mit dem graphischen Userinterface sind denkbar. Insbesondere bedeutet Verändern der Messzeitpunkte, dass der einem Messzeitpunkt zugeordnete Zeitpunkt, der beispielweise dem Messstart und dem Messende der Teilangiographiemessung entspricht, sich verändert. Der Vorteil der Veränderung der Messzeitpunkte auf dem graphischen Userinterface ist die einfache Handhabung und Anpassung der Teilangiographiemessungen durch den Benutzer.

Typischerweise weist jede Teilangiographiemessung Sequenzparameter auf. Die Sequenzparameter werden erfindungsgemäß automatisch festgelegt. Beispielsweise beeinflussen die anatomische Größe des Patienten, eine Größe und Ausrichtung der Teilmessbereiche und die zugeordnete Körperregion das automatische Festlegen der Sequenzparameter. Zweckmäßigerweise sind manche Sequenzparameter zur Durchführung der angiographischen Messung in Hinblick auf Kontrast, Signalstärke und Messzeitdauer angepasst. Die Sequenzparameter können üblicherweise mehr als einmal angepasst werden, vorzugsweise auch nach jedem Verändern der Messzeitpunkte derart, dass die Teilangiographiemessungen innerhalb der jeweiligen Messzeitpunkte durchführbar sind. Diese Anpassung erfolgt erfindungsgemäß automatisch und kann vorzugsweise automatisch im Hintergrund, d.h. nicht unmittelbar sichtbar für den Benutzer, erfolgen. Zweckmäßigerweise werden die Sequenzparameter der jeweiligen Teilangiographiemessung derart festgelegt, dass Teilangiographiebilder, die zur Erstellung der Angiographie benötigt sind, basierend auf den Teilangiographiemessungen ermittelt werden können. Beispielsweise kann bei einer Verlängerung der Messzeitdauer einer Teilangiographiemessung die Bildqualität erhöht werden, beispielsweise auf Kosten der Bildqualität einer anderen Teilangiographiemessung, deren Messzeitdauer verkürzt wird. Für die Bildqualität können unter anderem eine Auflösung und eine Schichtdicke der Teilangiographiemessung relevant sein.

Neben der oben beschriebenen direkten Veränderung der Messzeitpunkte der Teilangiographiemessungen durch den Benutzer können die Messzeitpunkte der Teilangiographiemessungen auch indirekt verändert werden, indem beispielsweise der Benutzer bestimmte Sequenzparameter der Teilangiographiemessungen verändert oder festlegt. Hierauf basierend werden einer oder mehrere Messzeitpunkte der Teilangiographiemessungen angepasst. Beispielsweise, wenn der Benutzer eine geringere Anzahl von zu messenden Schichten in einer Teilangiographiemessung festlegt, wird die Messzeitdauer der einen Teilangiographiemessung verkürzt und im gleichen Maße die Messzeitdauer der darauffolgenden und/oder der vorrausgehenden Teilangiographiemessung verlängert. Vorzugweise werden durch die Verlängerung der Messzeitdauer der darauffolgenden und/oder der vorausgehenden Teilangiographiemessung die Sequenzparameter automatisch festgelegt, wobei erfindungsgemäß immer ein automatisches Festlegen von Sequenzparametern der Teilangiographiemessungen basierend auf den veränderten Messstartzeiten und/oder Messendzeiten erfolgt.

Besonders vorteilhaft ist das erfindungsgemäße automatische Festlegen der Sequenzparameter, wobei für jeden Patienten eine hohe Bildqualität mit optimaler Abstimmung zwischen Injektion des Bolus und den Teilangiographiemessungen gewährleistet ist. Dadurch kann vermieden werden, dass Teilangiographiemessungen auf Grund nicht optimaler Festlegung der Sequenzparameter und des zeitlichen Ablaufs der angiographischen Messung wiederholt werden müssen. Eine Wiederholung kann sowohl zeitlich aufwändig als auch für den Patienten durch die erneute Gabe von Kontrastmittel risikobehaftet sein. Weiterhin können durch das automatische Festlegen der Sequenzparameter auch Benutzer mit weniger stark ausgeprägtem Wissen über Sequenzparameter oder sonstige Einstellungen verlässlich gute Angiographien der Patienten ermitteln. Alternativ können bestimmte Sequenzparameter auch unter Einhaltung bestimmter Vorgaben des Benutzers oder abgeleitet von der Patientenakte festgelegt werden. Beispielsweise kann der Benutzer frei und losgelöst von Vorgaben bestimmte Sequenzparameter festlegen.

Bestimmte Sequenzparameter einer Teilangiographiemessung können dabei unveränderbar gehalten werden. Dies können beispielsweise Sequenzparameter sein, die einen Kontrast der Teilangiographiebilder, die auf Basis der Teilangiographiemessungen ermittelt werden, beeinflussen. Andere Sequenzparameter der Teilangiographiemessung können variabel gestaltet werden. Dies können beispielsweise Sequenzparameter sein, die den Schichtabstand und die Auflösung beeinflussen. Die variabel gehaltenen Sequenzparameter werden erfindungsgemäß unter der Vorgabe der Randbedingung, dass die Teilangiographiemessung innerhalb der vorgegebenen Messzeitdauer abgeschlossen sein muss, automatisch ermittelt. Dies kann insbesondere unter der Vorgabe, eine möglichst hohe Bildqualität zu erhalten, geschehen.

Erfindungsgemäß werden die Sequenzparameter der Teilangiographiemessung der Steuereinheit der Magnetresonanzanlage insbesondere zur Durchführung der Teilangiographiemessungen bereitgestellt. Vorzugweise werden die Sequenzparameter der Steuereinheit automatisch bereitgestellt.

Zweckmäßigerweise werden die Teilangiographiemessungen automatisch durchgeführt, wobei die zugehörigen Teilmessbereiche jeweils im maximalen Gesichtsfeld der Anlage positioniert sind. Beispielsweise kann jede Teilangiographiemessung auch mehrmals, vorzugweise ohne Anpassung der Sequenzparameter durchgeführt werden. Bei der angiographischen Messung folgt üblicherweise einer ersten Durchführung der Teilangiographiemessungen eine zweite Durchführung der Teilangiographiemessungen. Insbesondere wird nach der ersten Durchführung der (pre-KM-)Teilangiographiemessungen und vor der zweiten Durchführung der (post-KM-)Teilangiographiemessungen der Kontrastmittelbolus injiziert. Beispielsweise kann die Injektion mittels eines Injektors durchgeführt werden. Der Zeitpunkt der Injektion kann typischerweise durch den Benutzer oder automatisiert im Ablauf der Teilangiographiemessungen festgelegt sein. Beispielsweise werden die Teilmessbereiche daher zweimal jeweils mit den pre-KM-Teilangiographiemessungen und den post-KM-Teilangiographiemessungen gemessen, wobei sich die Sequenzparameter der pre-KM-Teilangiographiemessungen und der post-KM-Teilangiographiemessungen vorzugsweise nicht unterscheiden. Daraus folgt, dass die Gesamtmessdauer der pre-KM-Teilangiographiemessungen vorzugsweise identisch zu der Gesamtmessdauer der post-KM-Teilangiographiemessungen ist.

Erfindungsgemäß wird die Angiographie anhand der durchgeführten Teilangiographiemessungen erstellt. Vorzugweise werden Teilangiographiebilder basierend auf den Teilangiographiemessungen ermittelt, um mittels der Teilangiographiebilder die Angiographie des Patienten zu erstellen. Hierfür vorteilhaft kann das räumliche Überlappen der Teilmessbereiche sein. Üblicherweise wird die Angiographie des Patienten durch die Differenz aus den pre-KM-Teilangiographiebildern und den post-KM-Teilangiographiebildern erstellt. Die Angiographie entspricht in diesem Fall im Wesentlichen lediglich einer Kontrastmitteldynamik innerhalb von Blutgefäßen des Patienten. Vorzugsweise wird die Angiographie basierend auf den Teilangiographiebildern der Teilangiographiemessung in den Teilmessbereichen automatisch mittels geeigneter Algorithmen erstellt. Alternativ kann auch der Benutzer die Teilangiographiebilder auf dem graphischen Userinterface separat betrachten oder manuell zu der Angiographie des Patienten zusammenfügen.

In einer Ausführungsform kann durch das Verändern der Messendzeit der nicht-letzten Teilangiographiemessung die Messstartzeit der nachfolgenden Teilangiographiemessung automatisch verändert werden. Beispielsweise wird durch das Verändern der Messendzeit der ersten Teilangiographiemessung die Messstartzeit der zweiten Teilangiographiemessung verändert. Durch das Verändern der Messendzeit der nicht-letzten Teilangiographiemessung kann auch lediglich die Messstartzeit der nachfolgenden Teilangiographiemessung automatisch verändert werden. Das Verändern der Messendzeit der nicht-letzten Teilangiographiemessung kann auch derart erfolgen, dass die Messstartzeit der nachfolgenden Teilangiographiemessung gemäß dem Unterschied zwischen der geänderten Messendzeit vor und nach der Veränderung verändert wird. Dies bedeutet insbesondere, dass die Gesamtmessdauer und die Summe der Messzeitdauer der Teilangiographiemessungen konstant bleiben.

In einer weiteren Ausführung kann durch das Verändern der Messstartzeit der nicht-ersten Teilangiographiemessung die Messendzeit der vorherigen Teilangiographiemessung automatisch verändert werden. Beispielsweise kann durch das Verändern der Messstartzeit der zweiten Teilangiographiemessung die Messendzeit der ersten Teilangiographiemessung verändert werden. Alternativ kann durch das Verändern der Messstartzeit der nicht-ersten Teilangiographiemessung lediglich die Messendzeit der vorherigen Teilangiographiemessung automatisch verändert werden. Das Verändern der Messstartzeit der nicht-ersten Teilangiographiemessung kann auch derart erfolgen, dass die Messendzeit der vorherigen Teilangiographiemessung gemäß dem Unterschied zwischen der geänderten Messstartzeit vor und nach der Veränderung verändert wird. Dies bedeutet insbesondere, dass die Gesamtmessdauer und die Summe der Messzeitdauer der Teilangiographiemessungen konstant bleiben.

In einer weiteren Ausführung werden die Messendzeiten aller Teilangiographiemessungen und die Messstartzeiten aller ausgenommen der ersten Teilangiographiemessung durch das Verändern der Messendzeit der letzten Teilangiographiemessung verändert. Dies bedeutet insbesondere, dass die Gesamtmessdauer gemäß der Veränderung der Messendzeit der letzten Teilangiographiemessung verändert wird. Vorzugweise wird durch das Verändern der Messendzeit der letzten Teilangiographiemessung insbesondere die Messzeitdauer aller Teilangiographiemessungen verändert und die Messzeitpausen, d.h. der Abstand zwischen der Messendzeit der vorherigen Teilangiographiemessung und der Messstartzeit der nachfolgenden Teilangiographiemessung, verbleiben unverändert. Beispielweise kann durch das Verändern der Messendzeit der letzten Teilangiographiemessung die Messzeitdauer aller Teilangiographiemessungen relativ zu ihrem Anteil an der Gesamtmessdauer oder proportional gemäß einer Gewichtung nach der Messzeitdauer der Teilangiographiemessungen verändert werden. Des Weiteren kann die Messzeitdauer der Teilangiographiemessung auch basierend auf Gewichtungen, die nicht auf der Messzeitdauer der Teilangiographiemessungen, sondern auf benutzerdefinierten oder automatisch vorgegebenen Faktoren basieren, erfolgen.

In einer weiteren Ausführung werden durch das Verändern der Messstartzeit der ersten Teilangiographiemessung, die Messstartzeiten und die Messendzeiten aller Teilangiographiemessungen automatisch verändert, insbesondere ohne Änderung der Gesamtmessdauer und der relativen Messzeitdauern zueinander. Vorzugsweise werden die Messstartzeiten und die Messendzeiten aller Teilangiographiemessungen - damit auch die Messzeitpausen - automatisch in dem Maße der Veränderung der Messstartzeit der ersten Teilangiographiemessung verändert. Beispielsweise folgt daraus eine Verschiebung aller Messzeitpunkte der Teilangiographiemessung um die gleiche Zeit. Durch das Verändern der Messstartzeit der ersten Teilangiographiemessung werden insbesondere Messzeitpausen zwischen den Teilangiographiemessungen konstant gehalten.

In einer Ausführungsform erfolgt durch das gleichzeitige Verändern der Messstartzeit und der Messendzeit einer Teilangiographiemessung oder der Messendzeit einer vorherigen Teilangiographiemessung und der Messstartzeit einer nachfolgenden Teilangiographiemessung, eine Veränderung der Messstartzeit und der Messendzeit aller Teilangiographiemessungen. Das gleichzeitige Verändern der Messendzeit einer vorherigen Teilangiographiemessung und der Messstartzeit einer nachfolgenden Teilangiographiemessung entspricht insbesondere einer Verschiebung der Messzeitpause. Zwei Messzeitpunkte können beispielsweise gleichzeitig per "Drag-and-Drop" verändert werden, vorzugweise derart, dass ein Bereich, der zwischen einer vorherigen Messendzeit und einer nachfolgenden Messstartzeit oder zwischen einer vorherigen Messstartzeit und einer nachfolgenden Messendzeit liegt, verschoben wird. Beispielweise kann durch das Verschieben einer Messbox einer Teilangiographiemessung, was dem gleichzeitigen Verschieben einer vorherigen Messstartzeit und einer nachfolgenden Messendzeit entspricht, entlang des Zeitstrahls die Messstartzeit und die Messendzeit der zugehörigen Teilangiographiemessung und alle Messzeitpunkte der anderen Teilangiographiemessungen gleichzeitig im gleichen Maße automatisch verschoben werden, so dass die Gesamtmessdauer und der Anteil der Messzeitpausen an der Gesamtmessdauer konstant bleibt.

Eine Ausführungsform sieht vor, dass der Körperbereich mindestens die Körperregionen Abdomen, Oberschenkel und Unterschenkel umfasst. Insbesondere kann der Körperbereich auch die Körperregion Nieren umfassen, wobei üblicherweise die Körperregion Abdomen auch die Nieren abdeckt. Vorzugweise wird der Körperbereich in mindestens einen ersten Teilmessbereich Abdomen, einen zweiten Messbereich Oberschenkel und einen dritten Teilmessbereich Unterschenkel mit jeweils einer ersten Teilangiographiemessung Abdomen, einer zweiten Teilangiographiemessung Oberschenkel und einer dritten Teilangiographiemessung Unterschenkel aufgeteilt. Üblicherweise erfassen die Teilmessbereiche zumindest einen Teil der zugeordneten Körperregionen, allerdings auch Teile von Körperregionen, denen der jeweilige Teilmessbereich nicht zugeordnet ist. Beispielsweise kann der erste Teilmessbereich Abdomen auch den Thorax oder der dritte Teilmessbereich auch die Füße umfassen. Alternativ kann der Körperbereich auch in mehrere, beispielsweise vier Teilmessbereiche mit einem zusätzlichen vierten Teilmessbereich Füße mit einer vierten zugeordneten Teilangiographiemessung Füße aufgeteilt werden. Die Anzahl der Teilmessbereiche und damit auch die Anzahl der Teilangiographiemessungen sind variabel und können patientenindividuell festgelegt und angezeigt werden.

In einer weiteren Ausführung umfassen die Messzeitpunkte der Teilangiographiemessungen vorzugweise je einen Mittelzeitpunkt, welcher Zeitpunkt einem Erfassen eines Zentrums eines k-Raums entspricht. Der Mittelzeitpunkt entspricht dem Zeitpunkt, bei dem die Teilangiographiemessungen vorzugweise je ein Zentrum des k-Raums erfassen. Insbesondere kann das Zentrum des k-Raums die niederfrequenten Anteile der Teilangiographiemessung enthalten im Vergleich zu den hochfrequenten Anteilen der Teilangiographiemessung im peripheren Teil des k-Raums. Das Zentrum des k-Raums ist daher vorzugsweise für den Kontrast, die Peripherie des k-Raums eher für die Details der Teilangiographiemessung verantwortlich. Beispielsweise wird ein Mittelzeitpunkt einer Teilangiographiemessung auf dem graphischen Userinterface angezeigt. Innerhalb der Messstartzeit und der Messendzeit der Teilangiographiemessungen kann der Mittelzeitpunkt verändert werden. Dies entspricht einer Veränderung auf dem graphischen Userinterface innerhalb der Grenzen der zugehörigen Messbox. Zweckmäßigerweise können solche Bereiche innerhalb der Messbox, welche mit einer Pulssequenz oder mit den im Hintergrund berechneten Sequenzparametern nicht realisiert werden können, automatisch ausgeschlossen werden. In einer weiteren Ausgestaltung könnte alternativ das Verändern des Mittelzeitpunkts in einen solchen Bereich nur unter bestimmten Vorgaben möglich sein, beispielsweise wäre dann das Verändern der anderen Messzeitpunkte erforderlich. Alternativ kann zumindest ein Mittelzeitpunkt angezeigt, aber nicht durch den Benutzer veränderbar sein.

In einer weiteren Ausführungsform kann zusätzlich die Kontrastmitteldynamik des Patienten auf dem graphischen Userinterface angezeigt werden. Die Kontrastmitteldynamik wird vorzugweise an zumindest einer Station einer Gefäßstruktur mittels einer Testbolusmessung erfasst, vorzugweise vor dem Aufteilen der angiographischen Messung in Teilangiographiemessungen. Die Gefäßstruktur umfasst zumindest teilweise die Blutgefäße des Patienten. Bei der Testbolusmessung handelt es sich um eine Serie kurzer Einzelmessungen, die es ermöglichen den zeitlichen Verlauf des Kontrastmittels zu erfassen. Mittels der Durchführung der Testbolusmessung, bei der vorzugweise ein Testbolus gespritzt wird, werden Testbolus-Kontrastmitteldynamikbilder erfasst, beispielweise auch mittels Algorithmen, die die Kontrastmitteldynamik hervorheben können oder dergleichen. Der Testbolus kann eine geringere Menge an Kontrastmittel im Vergleich zu dem Kontrastmittelbolus, der bei den darauffolgenden Teilangiographiemessungen injiziert wird, aufweisen. Von den Testbolus-Kontrastmitteldynamikbildern kann ein Kontrastmittelverlauf, insbesondere die Kontrastmitteldynamik abgeleitet werden.

Das Festlegen zumindest einer Station erfolgt üblicherweise entweder automatisch oder durch den Benutzer anhand der Auswertung der Testbolus-Kontrastmitteldynamikbilder. Beispielsweise berücksichtigt der Benutzer beim Festlegen der zumindest einen Station die Anatomie des Patienten. Vorzugweise kann die Kontrastmitteldynamik an zwei Stationen, wobei jeweils eine erste Station nahe bei der ersten Grenze und eine zweite Station nahe der zweiten Grenze des Körperbereichs ist, erfasst werden. Meist wird die erste Station den Abdomen des Patienten umfassen. Zweckmäßigerweise umfasst die zweite Station entweder den Unterschenkel oder bevorzugt die Füße, weil dort der Übergang zwischen arteriellen Blutgefäßen und venösen Blutgefäßen üblicherweise kürzer ist als im Abdomen. Die Kontrastmitteldynamik kann an zwei arteriellen Stationen, die beispielsweise automatisch oder durch den Benutzer festgelegt worden sind, erfasst werden.

Da üblicherweise die Gefäßstruktur sowohl arterielle Blutgefäße insbesondere Arterien als auch venöse Blutgefäße insbesondere Venen umfasst, kann die Kontrastmitteldynamik zumindest an einer arteriellen Station und einer venösen Station erfasst werden. Eine arterielle Station deckt bevorzugt zumindest ein arterielles Blutgefäß und eine venöse Station zumindest ein venöses Blutgefäß ab. Beispielsweise kann die erste Station der arteriellen Station und die zweite Station der venösen Station entsprechen. Anhand der Testbolusmessung kann vorzugweise an der ersten Station die arterielle Phase des injizierten Testbolus und an der zweiten Station die venöse Phase des injizierten Testbolus gemessen werden. Grundsätzlich ist allerdings auch möglich, dass an jeder Station sowohl die arterielle Phase als auch die venöse Phase oder lediglich die arterielle Phase oder lediglich die venöse Phase während der Testbolusmessung gemessen wird, wenn insbesondere eine Messzeitdauer und eine Messstartzeit der Testbolusmessung und der Zeitpunkt der Injektion des Testbolus entsprechend festgelegt sind.

Üblicherweise kann ein Teilmessbereich eine Station umfassen. Vorzugweise können daher die Messendzeiten der Teilangiographiemessungen derart festgelegt werden, dass die Teilangiographiemessungen vor Eintritt der venösen Phase des injizierten Bolus an den Stationen der Teilmessbereiche, die den jeweiligen Teilangiographiemessungen zugeordnet sind, durchführbar sind. Die Kontrastmitteldynamik kann insbesondere an der arteriellen Station in der arteriellen Phase des Testbolus und an der venösen Station in der arteriellen Phase des Testbolus erfasst werden. Dies ist insbesondere vorteilhaft, weil dadurch eine venöse Überlagerung der arteriellen Gefäße vermieden werden kann. Die venöse Überlagerung der arteriellen Gefäße kann eine Befundung anhand der angiographischen Messung erschweren und mitunter gänzlich verhindern, weil beispielweise für die Befundung insbesondere die arteriellen Gefäße bei der angiographischen Messung erfasst sein sollen.

In einer Ausführung wird die mittels der Testbolusmessung erfasste Kontrastmitteldynamik beim Aufteilen der angiographischen Messung in Teilangiographiemessungen berücksichtigt, weil üblicherweise sich die Kontrastmitteldynamik von Patient zu Patient unterscheidet. Außerdem kann die Kontrastmitteldynamik abhängig davon, welchen jeweiligen Teilmessbereich die Station umfasst, an der die Kontrastmitteldynamik erfasst wurde, bei demselben Patienten variieren. Beim Aufteilen der angiographischen Messung in Teilangiographiemessungen kann einer Teilangiographiemessung mit einer ersten von der Kontrastmitteldynamik abgeleiteten Kontrastmittelgeschwindigkeit eine geringere Messzeitdauer zugeordnet werden als einer Teilangiographiemessung mit einer zweiten, von der Kontrastmitteldynamik abgeleiteten langsameren Kontrastmittelgeschwindigkeit. Beispielsweise ist die erste Kontrastmittelgeschwindigkeit des Patienten höher als die zweite Kontrastmittelgeschwindigkeit eines weiteren Patienten einer anderen angiographischen Messung. In diesem Fall kann der Teilangiographiemessung des Patienten eine geringere Messzeitdauer zugeordnet werden als der Teilangiographiemessung des weiteren Patienten der anderen angiographischen Messung. In einem weiteren Beispiel unterscheidet sich die erste Kontrastmittelgeschwindigkeit des Patienten von der zweiten Kontrastmittelgeschwindigkeit des Patienten, wobei die erste Kontrastmittelgeschwindigkeit und die zweite Kontrastmittelgeschwindigkeit an Stationen in den jeweilig zugeordneten verschiedenen Teilmessbereichen erfasst wurden. Daher kann die Messzeitdauer der Teilangiographiemessungen abhängig von der jeweiligen Kontrastmittelgeschwindigkeit, welche in den zugeordneten verschiedenen Teilmessbereichen erfasst wurde, festgelegt werden.

Alternativ oder zusätzlich können auch andere von der Kontrastmitteldynamik abgeleitete Parameter wie z.B. ein Maximalwertzeitpunkt des Kontrastmittelverlaufs, der dem Zeitpunkt der maximalen Kontrastmittelanreicherung entspricht, oder eine Zirkulationszeit des Kontrastmittels für den Transport des Kontrastmittels berücksichtigt werden. Üblicherweise kann die Kontrastmittelgeschwindigkeit in die Zirkulationszeit des Kontrastmittels und die Zirkulationszeit des Kontrastmittels in die Kontrastmittelgeschwindigkeit umgerechnet werden.

Gemäß einer weiteren Ausführungsform können beispielsweise die Kontrastmitteldynamik, die Messzeitpunkte, insbesondere Messstartzeiten, die Messzeitdauer, die Messendzeiten und die Mittelzeitpunkte der Teilangiographiemessungen, auf dem graphischen Userinterface überlagert dargestellt werden, vorzugweise derart, dass die Messzeitpunkte einem Teil der Messbox und die Messzeitpause dem Zwischenraum zwischen den Messboxen entsprechen. Zusätzlich kann die Kontrastmitteldynamik bevorzugt derart dargestellt werden, dass für jede Station der Kontrastmittelverlauf dargestellt werden kann. Beispielsweise kann also der Kontrastmittelverlauf an der arteriellen Station und der venösen Station angezeigt werden. Alternativ oder zusätzlich kann der Kontrastmittelverlauf während der arteriellen Phase des Testbolus und der Kontrastmittelverlauf während der venösen Phase des Testbolus angezeigt werden. Üblicherweise ist es vorteilhaft, die Mittelzeitpunkte der Teilangiographiemessungen auf zumindest einen Maximalwertzeitpunkt abzustimmen. Dadurch kann bei der Durchführung der Teilangiographiemessungen ein hohes Signal und starker Kontrast sichergestellt sein.

In einer Ausführung können zusätzlich Symbole auf dem graphischen Userinterface entlang der Zeitachse beispielsweise als Indikator zum Atemanhalten und zum Weiteratmen angezeigt werden. Die Zeitpunkte dieser Symbole können durch den Benutzer verändert werden, insbesondere wenn der Benutzer die Messzeitpunkte der Teilangiographiemessungen festlegt. Üblicherweise sind die Zeitpunkte zum Atemanhalten und zum Weiteratmen abhängig von den Teilangiographiemessungen. Beim tatsächlichen Durchführen der Teilangiographiemessungen werden dann entsprechende Atemkommandos zu den Zeitpunkten ausgespielt.

Zusätzlich kann auch ein Symbol den Zeitpunkt der Kontrastmittelinjektion markieren. Der Zeitpunkt der Kontrastmittelinjektion ist üblicherweise bei t=0s definiert. Die Messzeitpunkte werden dann beim Durchführen der Teilangiographiemessungen auf den Zeitpunkt der Kontrastmittelinjektion bezogen ausgespielt. Insbesondere kann auch der Maximalwertzeitpunkt und ein Unterschied zwischen dem Maximalwertzeitpunkt und dem Mittelzeitpunkt der Teilangiographiemessungen angezeigt werden. Der Unterschied zwischen dem Maximalwertzeitpunkt und dem Mittelzeitpunkt entspricht insbesondere dem zeitlichen Abstand. Durch Eingabe eines numerischen Werts durch den Benutzer auf dem graphischen Userinterface kann der Unterschied zwischen zumindest einem Maximalwertzeitpunkt der Kontrastmitteldynamik und dem Mittelzeitpunkt der Teilangiographiemessungen angepasst werden. Es ist insbesondere vorteilhaft, wenn der Mittelzeitpunkt der Teilangiographiemessungen dahingehend verändert wird, dass der Abstand zwischen dem Maximalwertzeitpunkt und dem Mittelzeitpunkt der Teilangiographiemessungen verringert wird oder insbesondere dass der Mittelzeitpunkt der Teilangiographiemessung näher an der Messstartzeit als an der Messendzeit der jeweiligen Teilangiographiemessung ist.

In einer weiteren Ausgestaltung kann durch den Benutzer oder auch automatisch vorgegeben sein, was auf dem graphischen Userinterface angezeigt wird. Alternativ kann der Benutzer beispielsweise bestimmte Messzeiten teilweise aus- und einblenden.

Neben dem Verfahren betrifft die Erfindung auch eine Magnetresonanzanlage, die zur Durchführung des erfindungsgemäßen Verfahrens ausgebildet ist und in die eine Kontrastmittelinjektionsvorrichtung integriert ist. Die oben getätigten Ausführungen zum erfindungsgemäßen Verfahren lassen sich analog auf die Magnetresonanzanlage übertragen.

Die Magnetresonanzanlage ist somit dazu ausgelegt ein Verfahren zur Durchführung einer angiographischen Messung und Erstellung einer Angiographie von einem Körperbereich eines Patienten, wobei der Körperbereich größer als ein maximales Gesichtsfeld der Magnetresonanzanlage ist und die Magnetresonanzanlage eine Steuereinheit zur Steuerung der Durchführung der angiographischen Messung und der Erstellung der Angiographie aufweist, auszuführen.

Die Magnetresonanzanlage kann weitere Steuerungskomponenten aufweisen, welche zum Ausführen eines erfindungsgemäßen Verfahrens nötig und/oder vorteilhaft sind. Auf einer Speichereinheit der Planungseinheit und/oder der Steuereinheit und/oder der Messeinheit können Computerprogramme und weitere Software gespeichert sein, mittels derer ein Prozessor der Planungseinheit und/oder der Steuereinheit und/oder der Messeinheit einen Verfahrensablauf eines erfindungsgemäßen Verfahrens automatisch steuert und/oder ausführt.

Die Magnetresonanzanlage mit der Planungseinheit, der Steuereinheit und der Messeinheit ist dazu ausgelegt, dass die Durchführung der angiographischen Messung eines Körperbereichs mittels Teilangiographiemessungen erfolgt, wobei die mehreren Teilmessbereiche, die jeweils einer der Teilangiographiemessungen zugeordnet sind, Teile dieses einen Körperbereichs sind.

Die Magnetresonanzanlage mit der Planungseinheit, der Steuereinheit und der Messeinheit ist dazu ausgelegt, dass die Durchführung der angiographischen Messung eines Körperbereichs mittels Teilangiographiemessungen erfolgt, wobei die Messzeitpunkte der Teilangiographiemessungen derart verändert werden, dass bei der Veränderung eines Messzeitpunkts zumindest ein weiterer Messzeitpunkt automatisch verändert wird.

Die Magnetresonanzanlage mit der Planungseinheit, der Steuereinheit und der Messeinheit ist dazu ausgelegt, dass die Durchführung der angiographischen Messung eines Körperbereichs mittels Teilangiographiemessungen erfolgt, wobei die Messzeitpunkte der Teilangiographiemessungen automatisch festgelegt werden, vorzugsweise basierend auf einer anatomischen Größe und/oder einer Kontrastmitteldynamik des Patienten.

Weitere Vorteile und Einzelheiten der vorliegenden Erfindung ergeben sich aus den im Folgenden beschriebenen Ausführungsbeispielen sowie anhand der Zeichnung. Dabei zeigen:

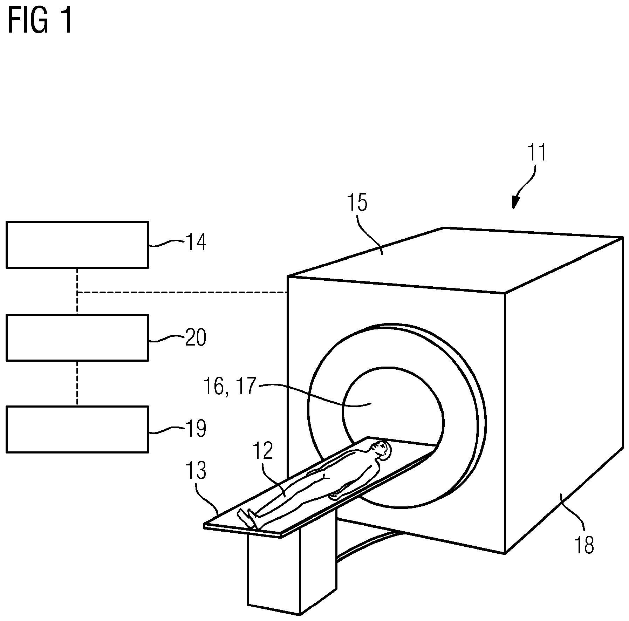

Fig. 1 eine erfindungsgemäße Magnetresonanzanlage in einer schematischen Darstellung
Fig. 2 einen Körperbereich mit einer oberen Grenze und einer unteren Grenze und mit Teilmessbereichen
Fig. 3 ein graphisches Userinterface unter anderem mit Messzeitpunkten und Messboxen von Teilangiographiemessungen, einer Kontrastmitteldynamik und einem Unterschied zwischen einem Maximalwertzeitpunkt und einem Mittelzeitpunkt einer ersten Teilangiographiemessung, einem Zeitstrahl, einem Zeitpunkt einer Kontrastmittelinjektion mit einem Symbol, je einem Symbol für den Zeitpunkt des Atemanhaltens und des Weiteratmens
Fig. 4 das graphische Userinterface nach Veränderung der Messendzeit der ersten Teilangiographiemessung im Vergleich zu Fig. 3
Fig. 5 das graphische Userinterface nach Veränderung der Messstartzeit der ersten Teilangiographiemessung im Vergleich zu Fig. 3
Fig. 6 das graphische Userinterface nach Veränderung der Messendzeit der letzten Teilangiographiemessung im Vergleich zu Fig. 3
Fig. 7 das Ablaufdiagramm einer angiographischen Messung ohne patientenindividuelle Anpassung, die nicht in den durch die Ansprüche definierten Schutzumfang der Erfindung fällt
Fig. 8 das Ablaufdiagramm einer angiographischen Messung mit Anpassung an einen Patienten von relativ kleiner Körpergröße
Fig. 9 das Ablaufdiagramm einer angiographischen Messung mit Anpassung an einen Patienten mit relativ hoher Zirkulationszeit des Kontrastmittels
Fig. 10 das Flussdiagramm eines erfindungsgemäßen Verfahrens Fig. 1 stellt eine erfindungsgemäße Magnetresonanzanlage 11 schematisch dar. Die Magnetresonanzanlage 11 umfasst eine von einer Magneteinheit 15 gebildeten Detektoreinheit mit einem Hauptmagneten zu einem Erzeugen eines starken und insbesondere konstanten Hauptmagnetfelds. Zudem weist das Magnetresonanzgerät 11 einen zylinderförmigen Patientenaufnahmebereich 16 zu einer Aufnahme eines Patienten 12 auf, wobei der Patientenaufnahmebereich 16 in einer Umfangsrichtung von der Magneteinheit 15 zylinderförmig umschlossen ist.

Im vorliegenden Fall kann der Patient 12 mittels einer Patientenlagerungsvorrichtung 13 der Magnetresonanzanlage 11 in den Patientenaufnahmebereich 16 der Magnetresonanzanlage 11 geschoben werden. Die Patientenlagerungsvorrichtung 13 weist hierzu einen Liegentisch auf, der bewegbar innerhalb der Magnetresonanzanlage 11 angeordnet ist. Der Patientenaufnahmebereich 16 der Magnetresonanzanlage 11 weist ein maximales Gesichtsfeld 17 mit einer Ausdehnung in Längsrichtung der Patientenlagerungsvorrichtung 13 der Magnetresonanzanlage 11 auf.

Die Magneteinheit 15 ist mittels einer Gehäuseverkleidung der Magnetresonanzanlage 11 nach außen abgeschirmt. Die Magneteinheit 13 weist weiterhin eine Gradientenspuleneinheit zu einer Erzeugung von Magnetfeldgradienten auf, die für eine Ortskodierung während einer Bildgebung verwendet werden. Die Gradientenspuleneinheit wird mittels einer Gradientensteuereinheit angesteuert. Des Weiteren weist die Magneteinheit eine Hochfrequenzantenneneinheit, welche im gezeigten Fall als fest in die Magnetresonanzanlage 11 integrierte Körperspule ausgebildet ist, und eine Hochfrequenzantennensteuereinheit zu einer Anregung einer Polarisation, die sich in dem von einem Hauptmagneten der Magneteinheit 15 erzeugten Hauptmagnetfeld einstellt, auf. Die Hochfrequenzantenneneinheit wird von der Hochfrequenzantennensteuereinheit angesteuert und strahlt hochfrequente Magnetresonanz-Sequenzen in einen Untersuchungsraum, der im Wesentlichen von dem Patientenaufnahmebereich 16 gebildet ist, ein. Die Hochfrequenzantenneneinheit ist weiterhin zum Empfang von Magnetresonanz-Signalen, insbesondere aus dem Patienten 12, ausgebildet.

Die Magnetresonanzanlage 11 weist weiterhin eine Messeinheit 18 auf. Zum Beispiel kann die Messeinheit 18 Teil der Magneteinheit 15 sein. Die Messeinheit 18 kann die Gradientenspuleneinheit und/oder die Hochfrequenzantenneneinheit aufweisen.

Zu einer Steuerung der Magneteinheit 15 weist die Magnetresonanzanlage 11 eine Steuereinheit 14 auf. Die Steuereinheit 14 steuert zentral die Magnetresonanzanlage 11, wie beispielsweise das Durchführen einer vorbestimmten bildgebenden Gradientenechosequenz.

Steuerinformationen wie beispielsweise Sequenzparameter, sowie rekonstruierte Magnetresonanz-Bilder können auf einer Anzeigeeinheit 19, beispielsweise auf zumindest einem Monitor, der Magnetresonanzanlage 11 für einen Benutzer angezeigt werden. Zudem können Steuerinformationen zwischen der Steuereinheit 19 und einer Planungseinheit 20 ausgetauscht werden. Typischerweise umfasst die Planungseinheit 20 die Anzeigeeinheit 19. Beispielsweise kann die Steuereinheit 14 Sequenzparameter der Planungseinheit zur Verfügung stellen, damit die Planungseinheit 20 diese Sequenzparameter auf zumindest einem Monitor für den Benutzer angezeigt werden. In einem weiteren Schritt kann die Planungseinheit 20 beispielsweise nach Änderung von zumindest einem Messzeitpunkt der Teilangiographiemessungen durch den Benutzer im Hintergrund Sequenzparameter festlegen und diese der Steuereinheit 14 bereitstellen. Mittels der Planungseinheit 20 können Informationen und/oder Sequenzparameter während eines Messvorgangs von einem Benutzer eingegeben werde.

Die dargestellte Magnetresonanzanlage 11 kann selbstverständlich weitere Komponenten umfassen, die Magnetresonanzanlagen 11 gewöhnlich aufweisen, beispielsweise einen Injektor zur Injektion des Kontrastmittels. Ebenso können die einzelnen Komponenten, insbesondere die Anzeigeeinheit 19, die Steuereinheit 14, die Messeinheit 18 und die Planungseinheit 20 in anderer Beziehung zueinander stehen und/oder in eine übergeordnete Einheit integriert sein. Eine allgemeine Funktionsweise einer Magnetresonanzanlage 11 ist zudem dem Fachmann bekannt, so dass auf eine detaillierte Beschreibung der weiteren Komponenten verzichtet wird.

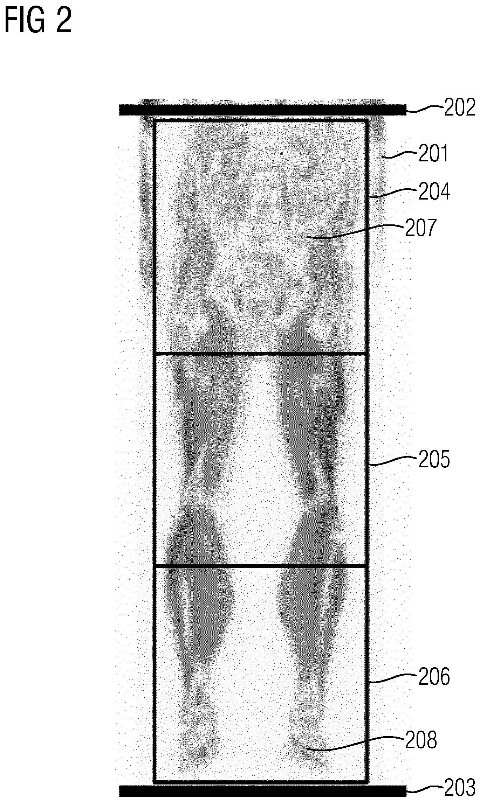

Das Aufteilen des Körperbereichs 201 in Teilmessbereiche ist exemplarisch in Fig. 2 illustriert. Der Körperbereich 201 weist eine obere Grenze 202 und eine untere Grenze 203 des Patienten 12 auf. Im Körperbereich 201 des Patienten 12 soll beispielsweise eine angiographische Messung durchgeführt werden, wobei das maximale Gesichtsfeld 17 der Magnetresonanzanlage 11 kleiner ist als der Körperbereich 201. Das bedeutet insbesondere, dass die Ausdehnung des Körperbereichs 201 in Längsrichtung der Patientenlagerungsvorrichtung 13 größer ist als die Ausdehnung des maximalen Gesichtsfelds 17 in Längsrichtung der Patientenlagerungsvorrichtung 13. Daher wird der Körperbereich 201 gleichmäßig in einen ersten Teilmessbereich 204, einen zweiten Teilmessbereich 205 und einen dritten Teilmessbereich 206 aufgeteilt, vorzugweise automatisch durch die Steuereinheit 14. Anders als in Fig. 2 gezeigt können die Teilmessbereiche 204, 205 und 206 sich teilweise überlappen. Der erste Teilmessbereich 204, der zweite Teilmessbereich 205 und der dritte Teilmessbereich 206 umfassen jeweils ein Volumen, das bei entsprechender Positionierung des Patienten 12 auf der Patientenlagerungsvorrichtung 13 oder bei entsprechenden Verfahren der Patientenlagerungsvorrichtung 13 mit dem Patienten 12 im maximalen Gesichtsfeld 17 der Magnetresonanzanlage 11 liegt. Außerdem sind eine erste Station 207 und eine zweite Station 208, an der jeweils eine erste Kontrastmitteldynamik 307 und eine zweite Kontrastmitteldynamik 308 erfasst wird, gezeigt.

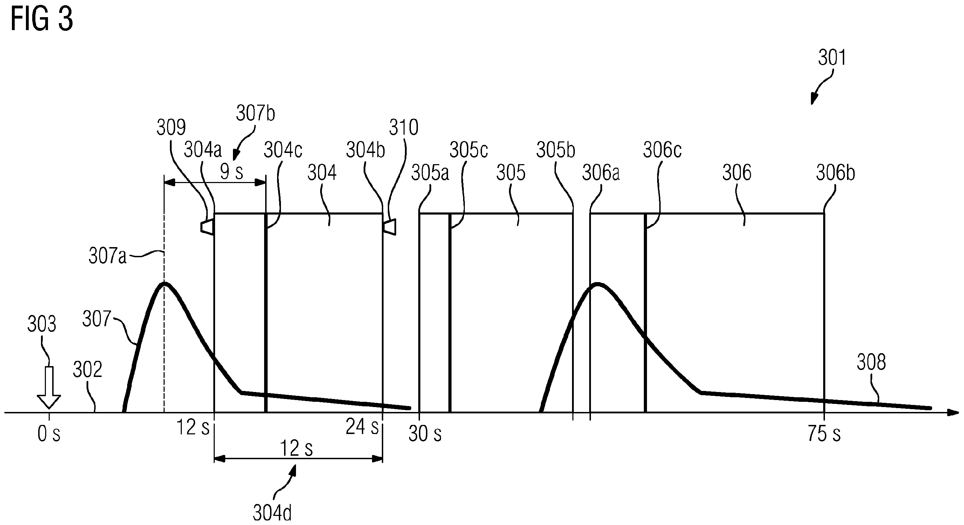

Fig. 3 stellt einen Ausschnitt auf einem graphischen Userinterface 301 für den Benutzer dar. Das graphische Userinterface 301 ist beispielsweise Teil der Anzeigeeinheit 19. Jedem Teilmessbereich ist je eine Teilangiographiemessung zugeordnet: dem ersten Teilmessbereich 204, eine erste Teilangiographiemessung 304, dem zweiten Teilmessbereich 205, eine zweite Teilangiographiemessung 305 und dem dritten Teilmessbereich 206, eine dritte Teilangiographiemessung 306. Jede Teilangiographiemessung ist mit je einer Messbox auf einem Zeitstrahl 302, welche beispielsweise als horizontale Zeitachse ausgebildet ist, dargestellt.

Die Messzeitpunkte der Teilangiographiemessungen können beispielsweise mit numerischen Werten auf dem graphischen Userinterface 301 angezeigt werden. Hierfür bietet es sich an, nicht absolute Zeiten anzugeben, sondern ausgewählte Messzeitpunkte auf einen Bezugszeitpunkt zu beziehen. Ein Bezugszeitpunkt kann beispielsweise der Zeitpunkt der Kontrastmittelgabe 303 sein. Zweckmäßigerweise kann dieser Zeitpunkt mit einem die Kontrastmittelgabe anzeigenden Symbol wie z.B. einem Schema einer Kontrastmittelspritze angezeigt werden. Die Messzeitdauer der Teilangiographiemessungen kann wahlweise auch durch einen Doppelpfeil symbolisiert dargestellt werden. Die Messboxen der Teilangiographiemessungen 304 - 306 werden durch die Paare aus der jeweiligen Messstartzeit und Messendzeit begrenzt. Zusätzlich können vorzugweise Messstartzeiten und Messendzeiten durch weitere Markierungen auf dem Zeitstrahl 302 durch beispielsweise gestrichelte Linien dargestellt werden.

304a zeigt eine Messstartzeit, 304b zeigt eine Messendzeit, 304c zeigt einen Mittelzeitpunkt und 304d zeigt eine Messzeitdauer der ersten Teilangiographiemessung 304 an.

305a zeigt eine Messstartzeit, 305b zeigt eine Messendzeit und 305c zeigt einen Mittelzeitpunkt der zweiten Teilangiographiemessung 305 an.

306a zeigt eine Messstartzeit, 306b zeigt eine Messendzeit und 306c zeigt einen Mittelzeitpunkt der dritten Teilangiographiemessung 306 an.

307 zeigt eine Kontrastmitteldynamik der ersten Station 207, 307a zeigt den Maximalwertzeitpunkt der Kontrastmitteldynamik der ersten Station 307, 307b zeigt einen Unterschied zwischen dem Maximalwertzeitpunkt der Kontrastmitteldynamik der ersten Station 307 und dem Mittelzeitpunkt der ersten Teilangiographiemessung 304c, und 308 eine Kontrastmitteldynamik der zweiten Station 208 an. Außerdem können verschiedene Symbole auf dem graphischen Userinterface 301 für den Benutzer dargestellt werden, beispielsweise der Zeitpunkt, an dem während der Durchführung der angiographischen Messung ein Befehl zum Atemanhalten 309 oder zum Weiteratmen 310 für den Patienten ausgespielt wird.

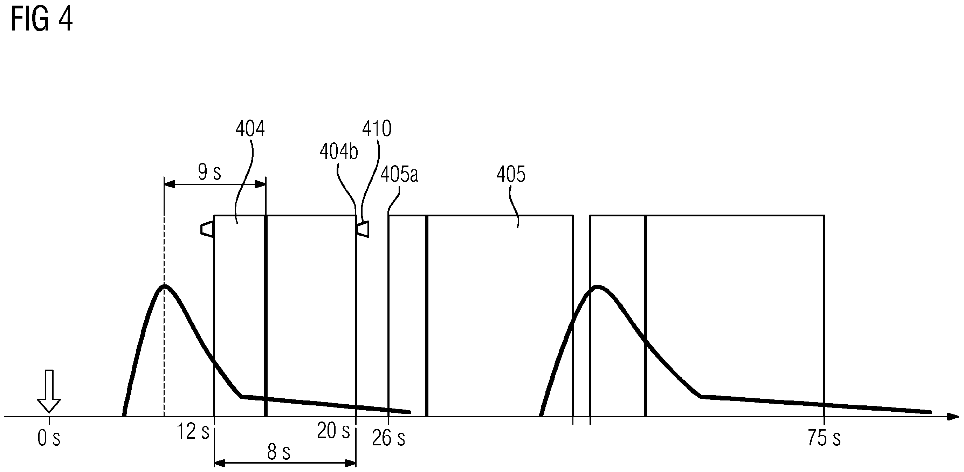

Anhand von Fig. 4 wird in einem nächsten Ausführungsbeispiel beispielsweise das Verändern der Messendzeit 304b der ersten Teilangiographiemessung 304, beispielsweise mittels Interaktion mit einem Cursor auf dem graphischen Userinterface 301 durch den Benutzer gezeigt. Aus Gründen der Übersichtlichkeit werden in erster Linie Bezugszeichen an den Merkmalen angezeigt, die sich im Vergleich zu Fig. 3 verändert haben. Erfindungsgemäß wird die Messstartzeit 305a der zweiten Teilangiographiemessung 305 automatisch verändert, insbesondere im gleichen Maße wie die Messendzeit 304b der ersten Teilangiographiemessung 304. Vorzugweise wird durch das Verändern der Messendzeit 304b der ersten Teilangiographiemessung 304 lediglich die Messstartzeit 305a der zweiten Teilangiographiemessung 305 automatisch verändert, insbesondere in dem gleichen Maße. Dadurch hält sich ein im Hintergrund ablaufendes automatisches Festlegen der Sequenzparameter der ersten Teilangiographiemessung 304 und der zweiten Teilangiographiemessung 305 voraussichtlich in einem geringeren Umfang, als wenn weitere Messzeitpunkte angepasst worden wären. Dies ist ferner besonders für den Benutzer, der leicht die Auswirkungen seiner Änderung überblicken kann, und für eine schnelle zeitliche Durchführung der angiographischen Messung vorteilhaft.

Fig. 4 zeigt daher die veränderte Messendzeit 404b der ersten Teilangiographiemessung 404 nach einer Veränderung beispielsweise durch den Nutzer per "Drag" bzw. per "Drag and Drop" und die daraufhin automatisch veränderte Messstartzeit 405a der zweiten Teilangiographiemessung 405. Die anderen Messstartzeiten verbleiben unverändert, wenn durch das Verändern der Messendzeit 304b der ersten Teilangiographiemessung 304 lediglich die Messstartzeit 305a der zweiten Teilangiographiemessung 305 automatisch verändert wird. Ferner wurde vorzugweise auch das Symbol für den Zeitpunkt zum Weiteratmen 410 verschoben. Da die Sequenzparameter auf eine Änderung hin erfindungsgemäß automatisch festgelegt werden, haben sich in Fig. 4 auch die Bezugszeichen der ersten Teilangiographiemessung und der zweiten Teilangiographiemessung verändert. In einer weiteren Ausführung ist es denkbar, dass ebenfalls die Mittelzeitpunkte 304c und 305c angepasst werden.

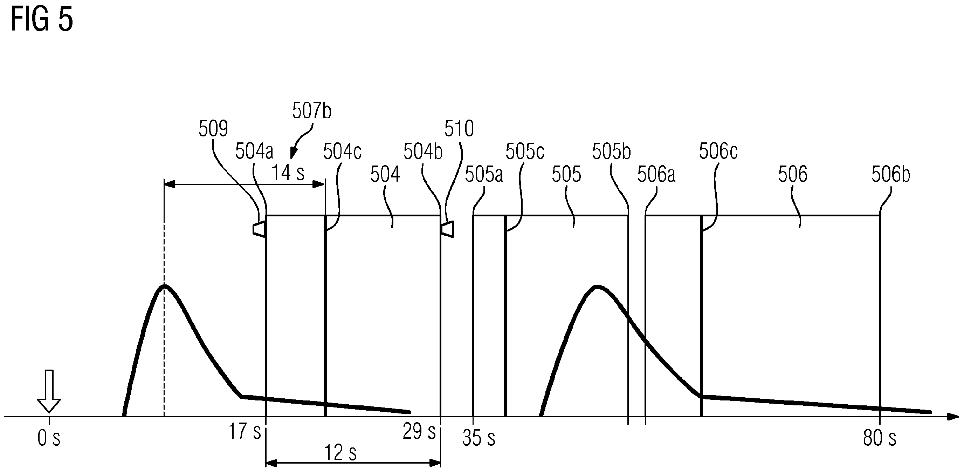

Anhand von Fig. 5 wird in einem weiteren Ausführungsbeispiel das Verändern der Messstartzeit 304a der ersten Teilangiographiemessung 304 gezeigt. Gemäß der Erfindung werden alle anderen Messzeitpunkte ebenfalls verändert. Bevorzugt erfolgt dies automatisch und die Veränderung aller Messzeitpunkte ist jeweils gleich, d.h. zum Beispiel eine Verschiebung aller Messzeitpunkte um 5s. Analog zum Verändern der Messstartzeit 304a der ersten Teilangiographiemessung 304 wäre beispielsweise das gleichzeitige Verändern der Messstartzeit 304a und Messendzeit 304b mittels Auswahl der Messstartbox der Teilangiographiemessung 304 oder das Verändern einer Messzeitpause, beispielsweise charakterisiert durch die Messendzeit 305b und die Messstartzeit 306a. Typischerweise erfolgt eine solche Interaktion durch den Benutzer mit einem Cursor auf dem graphischen Userinterface 301.

Fig. 5 stellt basierend auf dem Verändern der Messstartzeit 304a der ersten Teilangiographiemessung 304 um 5s alle Messzeitpunkte um 5s verschoben dar: die erste Teilangiographiemessung 504, die Messstartzeit 504a, die Messendzeit 504b und den Mittelzeitpunkt 504c der ersten Teilangiographiemessung 504 sowie die zweite Teilangiographiemessung 505, die Messstartzeit 505a, die Messendzeit 505b und den Mittelzeitpunkt 505c der zweiten Teilangiographiemessung 505 sowie die dritte Teilangiographiemessung 506, die Messstartzeit 506a, die Messendzeit 506b und den Mittelzeitpunkt 506c der dritten Teilangiographiemessung 506. Im Hintergrund wurden dementsprechend die Sequenzparameter aktualisiert. Außerdem wurde das Symbol für das Atemanhalten 509 und das Weiteratmen 510 verschoben. Außerdem hat sich dementsprechend der Unterschied 507b zwischen dem Maximalwertzeitpunkt der Kontrastmitteldynamik der ersten Station 307 und dem Mittelzeitpunkt der ersten Teilangiographiemessung 504c verändert.

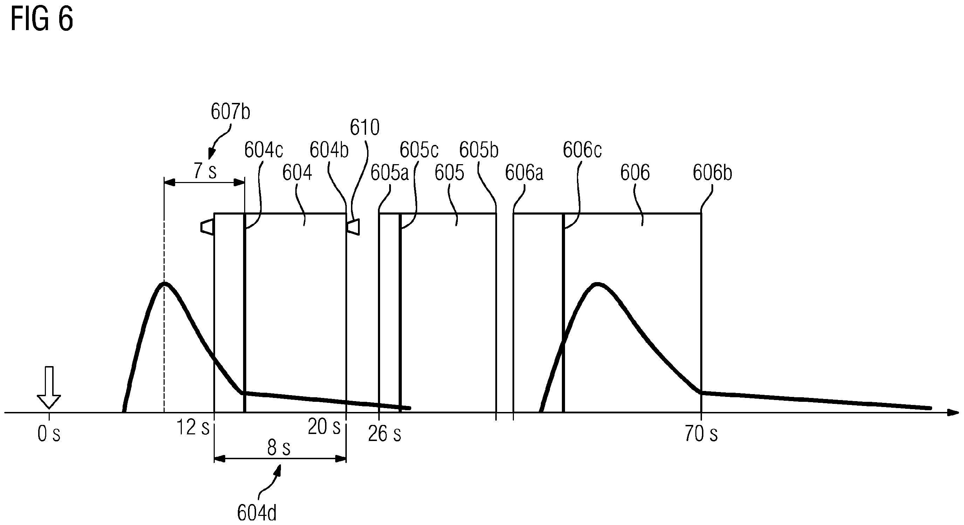

Anhand von Fig. 6 wird in einer weiteren erfindungsgemäßen Ausführung das Verändern der Messendzeit 306b der letzten Teilangiographiemessung 306 gezeigt. Dadurch werden automatisch die Messstartzeiten aller ausgenommen der ersten Teilangiographiemessungen und die Messendzeiten aller Teilangiographiemessungen verändert und zwar in der Art, dass die Messzeitpausen, charakterisiert durch 304b und 305a sowie 305b und 306a, nicht in ihrer Dauer, sondern nur die Position der Messzeitpausen verändert werden. Die Messzeitdauer der Teilangiographiemessungen hingegen, charakterisiert durch 304a und 304b, 305a und 305b sowie 306a und 306b, werden gemäß ihrem Anteil an der Gesamtmessdauer proportional verändert.

Besonders vorteilhaft ist die Kombination der Beispiele aus Fig. 5 und Fig. 6, weil durch das Verändern von lediglich zwei Messzeitpunkten durch den Benutzer, zunächst das Verändern der Messstartzeit der ersten Teilangiographiemessung und nachfolgend das Verändern der Messendzeit der letzten Teilangiographiemessung, die Messzeitpunkte aller Teilangiographiemessungen vorzugweise unter Berücksichtigung der Kontrastmitteldynamik festgelegt werden.

Fig. 6 stellt basierend auf dem Verändern der Messendzeit 306b der letzten Teilangiographiemessung 306 um -5s die konstanten Messzeitpausen und die anteilig zur Gesamtdauer verkürzten Teilangiographiemessungen dar: die erste Teilangiographiemessung 604, die Messendzeit 604b, den Mittelzeitpunkt 604c und die Messzeitdauer 604d der ersten Teilangiographiemessung 604 sowie die zweite Teilangiographiemessung 605, die Messstartzeit 605a, die Messendzeit 605b und den Mittelzeitpunkt 605c der zweiten Teilangiographiemessung 605 sowie die dritte Teilangiographiemessung 606, die Messstartzeit 606a, die Messendzeit 606b und den Mittelzeitpunkt 606c der dritten Teilangiographiemessung 606.

Die Mittelzeitpunkte der Teilangiographiemessungen sind ebenfalls automatisch verändert worden. Die Differenz zwischen 305a und 304b ist gleich zur Differenz aus 605a und 604b, ebenso die Differenz zwischen 306a und 305b zur Differenz aus 606a und 605b. Außerdem wurde das Symbol für das Weiteratmen 610 verschoben. Außerdem hat sich dementsprechend der Unterschied 607b zwischen dem Maximalwertzeitpunkt der Kontrastmitteldynamik der ersten Station 307 und dem Mittelzeitpunkt der ersten Teilangiographiemessung 604c verändert.

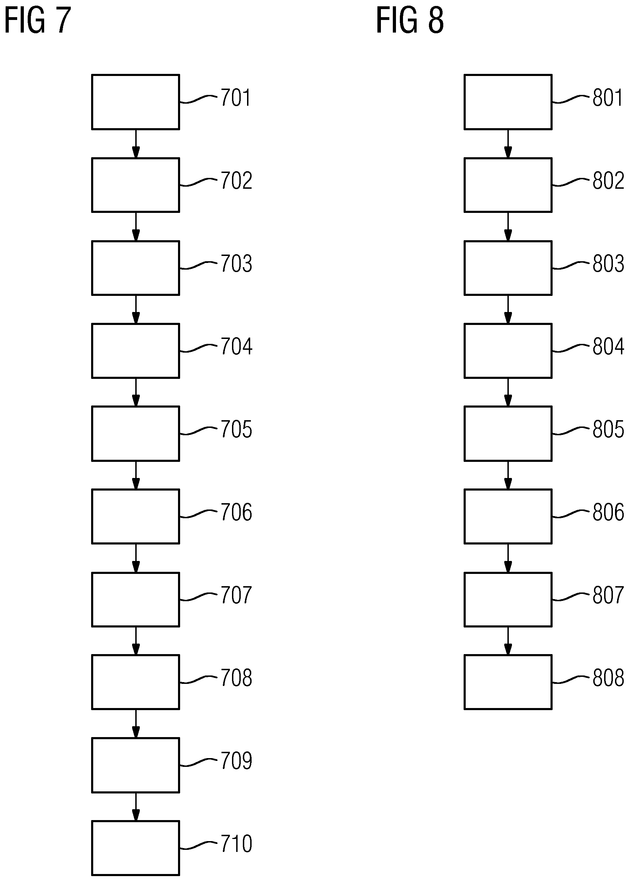

Ein beispielhaftes Verfahren, das nicht in den durch die Ansprüche definierten Schutzumfang der Erfindung fällt, ist in Fig. 7 gezeigt und entspricht einem Basisset als Startwert für eine angiographische Messung. D.h. üblicherweise wurde das Basisset noch nicht an patientenindividuelle Begebenheiten angepasst. Eine möglicherweise bei einer Testbolusmessung ermittelte Kontrastmitteldynamik und/oder eine anatomische Größe wie der Körpergröße des Patienten werden nicht weiter berücksichtigt. Ausgegangen wird von einer Aufteilung des Körperbereichs in vier Teilmessbereiche. In diesem Fall wird die angiographische Messung mittels einer Testbolusmessung 701, vier pre-KM-Teilangiographiemessungen 702-705, der Injektion des Bolus mit Kontrastmittel 706 und vier post-KM-Teilangiographiemessungen 707-710 durchgeführt. Je eine der vier pre-KM-Teilangiographiemessungen 702-705 und post-KM-Teilangiographiemessungen 707-710 ist jeweils einem Teilmessbereich Abdomen, Oberschenkel, Unterschenkel oder Fü-ße zugeordnet und weist eine Messzeitdauer auf. Die Gesamtmessdauer der pre-KM-Teilangiographiemessungen und der post-KM-Teilangiographiemessungen entspricht 68s. Im Detail kennzeichnen 701-710 folgende Schritte:
701 Testbolusmessung
702 Pre-KM-Teilangiographiemessung Abdomen, Messzeitdauer 18s
703 Pre-KM-Teilangiographiemessung Oberschenkel, Messzeitdauer 15s
704 Pre-KM-Teilangiographiemessung Unterschenkel, Messzeitdauer 15s
705 Pre-KM-Teilangiographiemessung Füße, Messzeitdauer 20s 706 Kontrastmittelinjektion
707 Post-KM-Teilangiographiemessung Abdomen, Messzeitdauer 18s
708 Post-KM-Teilangiographiemessung Oberschenkel, Messzeitdauer 15s
709 Post-KM-Teilangiographiemessung Unterschenkel, Messzeitdauer 15s
710 Post-KM-Teilangiographiemessung Füße, Messzeitdauer 20s

In einem weiteren Ausführungsbeispiel, welches in Fig. 8 illustriert ist, wird aufgrund der relativ kleinen Körpergröße der Körperbereich 201 des Patienten 12 im Vergleich zum Basisset in lediglich drei Teilmessbereiche aufgeteilt, wobei jedem Teilmessbereich je eine der pre-KM-Teilangiographiemessungen 802-804 und der post-KM-Teilangiographiemessungen 806-808 zugeordnet ist.

Unter Berücksichtigung der von der Kontrastmitteldynamik abgeleiteten Zirkulationszeit, die im Wesentlichen vergleichbar zu einem größeren Patienten ist, wird die Messzeitdauer der einzelnen Teilangiographiemessungen im Vergleich zum Basisset nicht verändert. Durch den Wegfall des vierten Teilmessbereichs und der Einsparung der Messzeitdauer der entsprechenden vierten Teilangiographiemessung, ist die Gesamtmessdauer der pre-KM-Teilangiographiemessungen und der post-KM-Teilangiographiemessungen zunächst auf 53s deutlich verkürzt. Der Benutzer verlängert in diesem Fall mittels entsprechenden Vergrößerns der Messendzeit der letzten Teilangiographiemessung Unterschenkel und Füße die Messzeitdauer aller Teilangiographiemessungen, bis die Gesamtmessdauer beispielsweise wieder 68s entspricht. Die dadurch jeder der drei Teilangiographiemessungen neu zur Verfügung stehende Messzeitdauer wird von der Steuereinheit für die Messung in einer höheren Auflösung oder von mehr Signal genutzt, im konkreten Beispiel kann in der Teilangiographiemessung Oberschenkel die Matrixauflösung von 256 auf 320 erhöht werden und in der Teilangiographiemessung Unterschenkel und Füße der iPAT-Faktor von 3 auf 2 verringert werden.

Im Detail kennzeichnen 801-808 folgende Schritte:
801 Testbolusmessung
802 Pre-KM-Teilangiographiemessung Abdomen, Messzeitdauer 23s
803 Pre-KM-Teilangiographiemessung Oberschenkel, Messzeitdauer 19s
804 Pre-KM-Teilangiographiemessung Unterschenkel und Füße, Messzeitdauer 26s
805 Kontrastmittelinjektion
806 Post-KM-Teilangiographiemessung Abdomen, Messzeitdauer 23s
807 Post-KM-Teilangiographiemessung Oberschenkel, Messzeitdauer 19s
808 Post-KM-Teilangiographiemessung Unterschenkel und Füße, Messzeitdauer 26s

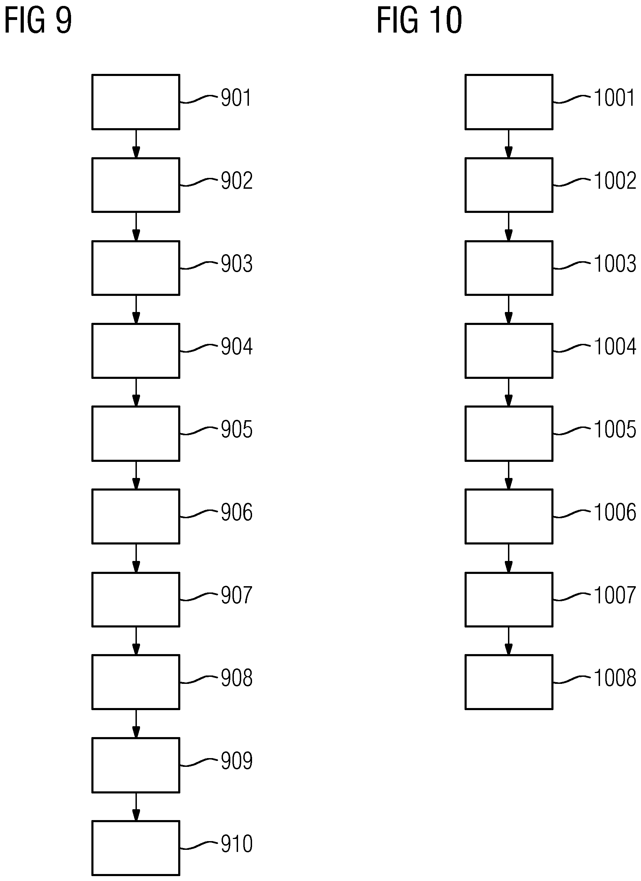

In diesem nächsten Ausführungsbeispiel hat der Patient 12 basierend auf der ermittelten Kontrastmitteldynamik der Testbolus-Messung eine schnelle Zirkulationszeit. Das entsprechende Ablaufdiagramm ist in Fig. 9 gezeigt. Die geplante Gesamtmessdauer der pre-KM-Teilangiographiemessungen und der post-KM-Teilangiographiemessungen des Basisset von 68s ist zu lange. Der Benutzer zieht die Messboxen kleiner: in diesem Fall werden die Sequenzparameter der Teilangiographiemessung derart angepasst, dass die Messzeitdauer eingehalten wird beispielsweise durch die Erhöhung des iPat-Faktors oder die Vergrößerung der Schichtdicke.

Im Detail kennzeichnen 901-910 folgende Schritte:
901 Testbolusmessung
902 Pre-KM-Teilangiographiemessung Abdomen, Messzeitdauer 15s
903 Pre-KM-Teilangiographiemessung Oberschenkel, Messzeitdauer 12s
904 Pre-KM-Teilangiographiemessung Unterschenkel, Messzeitdauer 12s
905 Pre-KM-Teilangiographiemessung Füße, Messzeitdauer 17s
906 Kontrastmittelinjektion
907 Post-KM-Teilangiographiemessung Abdomen, Messzeitdauer 15s
908 Post-KM-Teilangiographiemessung Oberschenkel, Messzeitdauer 12s
909 Post-KM-Teilangiographiemessung Unterschenkel, Messzeitdauer 12s
910 Post-KM-Teilangiographiemessung Füße, Messzeitdauer 17s

Fig. 10 zeigt ein Flussdiagramm eines erfindungsgemäßen Verfahrens. Das Verfahren umfasst die Verfahrensschritte 1001 - 1008, wobei bei der Beschreibung der Verfahrensschritte 1001 - 1008, auch Beschreibungsteile einschließlich der entsprechenden im Zusammenhang mit den anderen Figuren eingeführten Bezugszeichen verwendet werden.

Verfahrensschritt 1001 kennzeichnet das Erfassen einer anatomischen Größe des Patienten 12 und einer oberen Grenze 202 und einer unteren Grenze 203 des Körperbereichs 201.

Verfahrensschritt 1002 kennzeichnet das Aufteilen der angiographischen Messung in Teilangiographiemessungen, wobei die Steuereinheit 14 den Körperbereich 201 in mehrere, nacheinander zu messende Teilmessbereiche aufteilt,
wobei jeder Teilmessbereich unterschiedlichen Körperregionen zugeordnet ist,
wobei jeder Teilmessbereich nicht größer als das maximale Gesichtsfeld ist und je einer Teilangiographiemessung zugeordnet ist, und
wobei jeder Teilangiographiemessung Messzeitpunkte, umfassend jeweils eine Messstartzeit und eine Messendzeit, zugeordnet sind,
so dass eine Gesamtmessdauer der angiographischen Messung durch die Messstartzeit der ersten Teilangiographiemessung und die Messendzeit der letzten Teilangiographiemessung festgelegt ist.

Verfahrensschritt 1003 kennzeichnet das Anzeigen der Messstartzeiten, der Messzeitdauer und der Messendzeiten der Teilangiographiemessungen auf einem graphischen Userinterface.

Verfahrensschritt 1004 kennzeichnet das Verändern der Messzeitpunkte, wobei bei der Veränderung eines Messzeitpunkts einer Teilangiographiemessung automatisch eine Anpassung mindestens eines weiteren Messzeitpunkts einer anderen Teilangiographiemessung erfolgt,

Verfahrensschritt 1005 kennzeichnet das automatische Festlegen von Sequenzparametern der Teilangiographiemessungen basierend auf den veränderten Messstartzeiten und/oder Messendzeiten derart, dass die Teilangiographiemessung zwischen zugehöriger Messstartzeit und Messendzeit durchführbar ist, so dass die Teilangiographiemessung innerhalb der vorgegebenen Messzeitdauer abgeschlossen sein muss,
Verfahrensschritt 1006 kennzeichnet das Bereitstellen der Sequenzparameter der Steuereinheit 14 der Magnetresonanzanlage 11,
Verfahrensschritt 1007 kennzeichnet das Durchführen der Teilangiographiemessungen, und
Verfahrensschritt 1008 kennzeichnet das Erstellen der Angiographie anhand der durchgeführten Teilangiographiemessungen.

## Patentansprüche

1. Verfahren zur Durchführung einer angiographischen Messung und Erstellung einer Angiographie von einem Körperbereich eines Patienten in einer Magnetresonanzanlage, wobei der Körperbereich größer als ein maximales Gesichtsfeld der Magnetresonanzanlage ist und die Magnetresonanzanlage eine Steuereinheit zur Steuerung der Durchführung der angiographischen Messung und der Erstellung der Angiographie aufweist, mit den folgenden Schritten:
- Erfassen einer anatomischen Größe des Patienten und einer oberen Grenze und einer unteren Grenze des Körperbereichs,
- Aufteilen der angiographischen Messung in Teilangiographiemessungen, wobei die Steuereinheit den Körperbereich in mehrere, nacheinander zu messende Teilmessbereiche aufteilt,
wobei jeder Teilmessbereich unterschiedlichen Körperregionen zugeordnet ist,
wobei jeder Teilmessbereich nicht größer als das maximale Gesichtsfeld ist und je einer Teilangiographiemessung zugeordnet ist, und
wobei jeder Teilangiographiemessung Messzeitpunkte, umfassend jeweils eine Messstartzeit und eine Messendzeit, zugeordnet sind,
so dass eine Gesamtmessdauer der angiographischen Messung durch die Messstartzeit der ersten Teilangiographiemessung und die Messendzeit der letzten Teilangiographiemessung festgelegt ist,
- Anzeigen der Messstartzeiten, der Messzeitdauer und der Messendzeiten der Teilangiographiemessungen auf einem graphischen Userinterface,
- Verändern der Messzeitpunkte, wobei bei der Veränderung eines Messzeitpunkts einer Teilangiographiemessung automatisch eine Anpassung mindestens eines weiteren Messzeitpunkts einer anderen Teilangiographiemessung erfolgt,
- Automatisches Festlegen von Sequenzparametern der Teilangiographiemessungen basierend auf den veränderten Messstartzeiten und/oder Messendzeiten derart, dass die Teilangiographiemessung zwischen zugehöriger Messstartzeit und Messendzeit durchführbar ist, so dass die Teilangiographiemessung innerhalb der vorgegebenen Messzeitdauer abgeschlossen sein muss,
- Bereitstellen der Sequenzparameter der Steuereinheit der Magnetresonanzanlage,
- Durchführen der Teilangiographiemessungen, und
- Erstellen der Angiographie anhand der durchgeführten Teilangiographiemessungen.

2. Verfahren nach Anspruch 1, wobei durch das Verändern der Messendzeit der nicht-letzten Teilangiographiemessung der Messstartzeit der nachfolgenden Teilangiographiemessung automatisch verändert wird.

3. Verfahren nach einem der vorhergehenden Ansprüche, wobei durch das Verändern der Messendzeit der letzten Teilangiographiemessung, die Messendzeiten aller Teilangiographiemessungen und die Messstartzeiten aller ausgenommen der ersten Teilangiographiemessung verändert werden.

4. Verfahren nach Anspruch 3, wobei eine Messzeitpause, charakterisiert durch den Abstand der Messendzeit der vorherigen Teilangiographiemessung und der Messstartzeit der nachfolgenden Teilangiographiemessung, konstant bleibt und insbesondere die Messzeitdauer aller Teilangiographiemessungen relativ zu ihrem Anteil an der Gesamtmessdauer verändert wird.

5. Verfahren nach einem der vorhergehenden Ansprüche, wobei durch das Verändern der Messstartzeit der nicht-ersten Teilangiographiemessung die Messendzeit der vorherigen Teilangiographiemessung automatisch verändert wird.

6. Verfahren nach einem der vorhergehenden Ansprüche, wobei durch das Verändern der Messstartzeit der ersten Teilangiographiemessung, die Messstartzeiten und die Messendzeiten aller Teilangiographiemessungen automatisch verändert werden, insbesondere ohne Änderung der Gesamtmessdauer und der relativen Messzeitdauern zueinander.

7. Verfahren nach einem der vorhergehenden Ansprüche, wobei durch das gleichzeitige Verändern der Messstartzeit und der Messendzeit einer Teilangiographiemessung oder der Messendzeit einer vorherigen Teilangiographiemessung und der Messstartzeit einer nachfolgenden Teilangiographiemessung, eine Veränderung der Messstartzeit und der Messendzeit aller Teilangiographiemessungen erfolgt.

8. Verfahren nach einem der vorhergehenden Ansprüche, wobei die Messzeitpunkte der Teilangiographiemessungen zusätzlich jeweils einen Mittelzeitpunkt umfassen, welcher dem Zeitpunkt des Erfassens eines Zentrums eines k-Raums entspricht, wobei der Mittelzeitpunkt auf dem graphischen Userinterface angezeigt wird.

9. Verfahren nach Anspruch 8, wobei innerhalb der Messstartzeit und der Messendzeit der Teilangiographiemessungen der Mittelzeitpunkt verändert wird.

10. Verfahren nach einem der vorhergehenden Ansprüche, wobei die obere Grenze und die untere Grenze des Körperbereichs automatisch mittels Erkennung von Landmarken erfasst werden.

11. Verfahren nach einem der vorhergehenden Ansprüche, wobei der Körperbereich mindestens die Körperregionen Abdomen, Oberschenkel und Unterschenkel umfasst und in mindestens drei Teilmessbereiche, einem ersten Teilmessbereich Abdomen, einem zweiten Teilmessbereich Oberschenkel und einem dritten Teilmessbereich Unterschenkel, aufgeteilt wird.

12. Verfahren nach einem der vorhergehenden Ansprüche, wobei eine Kontrastmitteldynamik an zumindest einer Station des Körperbereichs, insbesondere an zwei Stationen des Körperbereichs, mittels einer Testbolusmessung mit einem injizierten Testbolus vor dem Aufteilen der angiographischen Messung in Teilangiographiemessungen erfasst wird.

13. Verfahren nach Anspruch 12, wobei eine Kontrastmitteldynamik an zwei Stationen des Körperbereichs erfasst wird, und wobei mindestens eine Station die Füße oder die Unterschenkel des Patienten umfasst.

14. Verfahren nach Anspruch 12 oder 13, wobei die Kontrastmitteldynamik und die Messstartzeiten, die Messzeitdauer und die Messendzeiten der Teilangiographiemessungen auf dem graphischen Userinterface überlagert dargestellt wird, und/oder wobei die Kontrastmitteldynamik beim Aufteilen der angiographischen Messung in Teilangiographiemessungen verwendet wird, indem einer Teilangiographiemessung mit einer ersten von der Kontrastmitteldynamik abgeleiteten Kontrastmittelgeschwindigkeit eine geringere Messzeitdauer zugeordnet wird als einer Teilangiographiemessung mit einer zweiten, langsameren von der Kontrastmitteldynamik abgeleiteten Kontrastmittelgeschwindigkeit.

15. Magnetresonanzanlage, ausgebildet zur Durchführung des Verfahrens nach einem der vorangehenden Ansprüche, aufweisend:
- eine Planungseinheit, welche ausgebildet ist:
- zum Erfassen einer anatomischen Größe des Patienten und einer oberen Grenze und einer unteren Grenze des Körperbereichs,
- zum Aufteilen der angiographischen Messung in Teilangiographiemessungen, wodurch der Körperbereich in mehrere, nacheinander zu messende Teilmessbereiche aufgeteilt wird,
wobei jeder Teilmessbereich unterschiedlichen Körperregionen zugeordnet ist,
wobei jeder Teilmessbereich nicht größer als das maximale Gesichtsfeld ist und je einer Teilangiographiemessung zugeordnet ist, und
wobei jeder Teilangiographiemessung Messzeitpunkte, umfassend jeweils eine Messstartzeit und eine Messendzeit, zugeordnet sind,
so dass eine Gesamtmessdauer der angiographischen Messung durch die Messstartzeit der ersten Teilangiographiemessung und die Messendzeit der letzten Teilangiographiemessung festgelegt ist,
- zum Verändern der Messzeitpunkte, wobei bei der Veränderung eines Messzeitpunkts einer Teilangiographiemessung automatisch eine Anpassung mindestens eines weiteren Messzeitpunkts einer anderen Teilangiographiemessung erfolgt,
- zum automatischen Festlegen von Sequenzparametern der Teilangiographiemessungen basierend auf den veränderten Messstartzeiten und/oder Messendzeiten derart, dass die Teilangiographiemessung zwischen zugehöriger Messstartzeit und Messendzeit durchführbar ist, so dass die Teilangiographiemessung innerhalb der vorgegebenen Messzeitdauer abgeschlossen sein muss,
- zum Bereitstellen der Sequenzparameter einer Steuereinheit,
- zum Anzeigen der Messstartzeiten, der Messzeitdauer und der Messendzeiten der Teilangiographiemessungen auf einem graphischen Userinterface und
- die Steuereinheit zum Steuern der Magnetresonanzanlage und einer Messeinheit, basierend auf den bereitgestellten Sequenzparametern und
- die Messeinheit zum Durchführen der Teilangiographiemessungen und zum Erstellen der Angiographie anhand der durchgeführten Teilangiographiemessungen.

## Claims

1. Method for performing an angiographic measurement and creating an angiogram of a body region of a patient in a magnetic-resonance system, wherein the body region is larger than a maximum field of view of the magnetic-resonance system and the magnetic-resonance system comprises a control unit for controlling the performance of the angiographic measurement and the creation of the angiogram with the following steps:
- acquisition of an anatomical size of the patient and an upper limit and a lower limit of the body region,
- division of the angiographic measurement into partial angiographic measurements, wherein the control unit divides the body region into a plurality of partial measuring ranges to be measured in succession,
wherein each partial measuring range is assigned to different body regions,
wherein each partial measuring range is no larger than the maximum field of view and each is assigned to a partial angiographic measurement, and
wherein each partial angiographic measurement is assigned measuring time points, including in each case a measurement start time and a measurement end time,
so that an overall measurement duration of the angiographic measurement is defined by the measurement start time of the first partial angiographic measurement and the measurement end time of the last partial angiographic measurement,
- displaying the measurement start times, the measurement duration and the measurement end times of the partial angiographic measurements on a graphical user interface,
- changing the measuring time points, wherein, when a measuring time point of a partial angiographic measurement is changed, an adaptation of at least one further measuring time point of another partial angiographic measurement is performed automatically,
- automatic definition of sequence parameters of the partial angiographic measurements based on the changed measurement start times and/or measurement end times such that the partial angiographic measurement can be performed between the associated measurement start time and measurement end time, so that the partial angiographic measurement must be completed within the specified measurement duration,
- provision of the sequence parameters of the control unit of the magnetic-resonance system,
- performance of the partial angiographic measurements, and
- creation of the angiogram using the partial angiographic measurements performed.

2. Method according to claim 1, wherein changing the measurement end time of the non-last partial angiographic measurement causes the measurement start time of the subsequent partial angiographic measurement to be changed automatically.

3. Method according to one of the preceding claims, wherein changing the measurement end time of the last partial angiographic measurement causes the measurement end times of all partial angiographic measurements and the measurement start times of all except for the first partial angiographic measurement to be changed.

4. Method according to claim 3, wherein a measurement time pause, **characterised by** the interval between the measurement end time of the preceding partial angiographic measurement and the measurement start time of the subsequent partial angiographic measurement, remains constant and in particular the measurement duration of all partial angiographic measurements relative to their proportional share in the overall measurement duration is changed.

5. Method according to one of the preceding claims, wherein changing the measurement start time of the non-first partial angiographic measurement causes the measurement end time of the preceding partial angiographic measurement to be changed automatically.

6. Method according to one of the preceding claims, wherein changing the measurement start time of the first partial angiographic measurement causes the measurement start times and the measurement end times of all partial angiographic measurements to be changed automatically, in particular without changing the overall measurement duration and the measurement durations relative to one another.

7. Method according to one of the preceding claims, wherein simultaneously changing the measurement start time and the measurement end time of a partial angiographic measurement or the measurement end time of a preceding partial angiographic measurement and the measurement start time of a subsequent partial angiographic measurement causes a change to the measurement start time and the measurement end time of all partial angiographic measurements.

8. Method according to one of the preceding claims, wherein the measuring time points of the partial angiographic measurements additionally in each case include a mid-time point, which corresponds to the time point of the acquisition of a centre of a k-space, wherein the mid-time point is displayed on the graphical user interface.

9. Method according to claim 8, wherein the mid-time point is changed between the measurement start time and the measurement end time of the partial angiographic measurements.

10. Method according to one of the preceding claims, wherein the upper limit and the lower limit of the body region are automatically acquired by means of the recognition of landmarks.

11. Method according to one of the preceding claims, wherein the body region includes at least the body regions 'abdomen', 'thigh' and 'lower leg' and is divided into at least three partial measuring ranges, a first partial measuring range 'abdomen', a second partial measuring range `thigh' and a third partial measuring range 'lower leg'.

12. Method according to one of the preceding claims, wherein contrast medium dynamics are acquired on at least one station of the body region, in particular on two stations of the body region, by means of a test bolus measurement with an injected test bolus before the division of the angiographic measurement in partial angiographic measurements.

13. Method according to claim 12, wherein contrast medium dynamics are acquired on two stations of the body region, and wherein at least one station includes the feet or the lower leg of the patient.

14. Method according to claim 12 or 13, wherein the contrast medium dynamics and the measurement start times, the measurement duration and the measurement end times of the partial angiographic measurements are depicted superimposed on the graphical user interface, and/or
wherein the contrast medium dynamics on the division of the angiographic measurement into partial angiographic measurements are used in that a partial angiographic measurement with a first contrast medium velocity derived from the contrast medium dynamics is assigned a shorter measurement duration than a partial angiographic measurement with a second, slower, contrast medium velocity derived from the contrast medium dynamics.

15. Magnetic-resonance system embodied to perform the method according to one of the preceding claims, having:
- a planning unit embodied:
- to acquire an anatomical size of the patient and an upper limit and a lower limit of the body region,
- to divide the angiographic measurement into partial angiographic measurements as a result of which the body region is divided into a plurality of partial measuring ranges to be measured in succession,
wherein each partial measuring range is assigned to different body regions,
wherein each partial measuring range is no larger than the maximum field of view and each is assigned to partial angiographic measurement, and
wherein each partial angiographic measurement is assigned measuring time points, including in each case a measurement start time and a measurement end time,
so that an overall measurement duration of the angiographic measurement is defined by the measurement start time of the first partial angiographic measurement and the measurement end time of the last partial angiographic measurement,
- to change the measuring time points, wherein, when a measuring time point of a partial angiographic measurement is changed, an adaptation of at least one further measuring time point of another partial angiographic measurement is performed automatically,
- to automatically define sequence parameters of the partial angiographic measurements based on the changed measurement start times and/or measurement end times such that the partial angiographic measurement can be performed between the associated measurement start time and measurement end time, so that the partial angiographic measurement must be completed within the specified measurement duration,
- to provide the sequence parameters of a control unit,
- to display the measurement start times, the measurement duration and the measurement end times of the partial angiographic measurements on a graphical user interface and
- to change the measuring time points, wherein, when a measuring time point of a partial angiographic measurement is changed, adaptation of at least one further measuring time point of another partial angiographic measurement is performed automatically,
- the control unit for controlling the magnetic-resonance system and a measuring unit based on the sequence parameters provided and
- the measuring unit for performing the partial angiographic measurements and creating the angiogram using the partial angiographic measurements performed.

## Revendications

1. Procédé d'exécution d'une mesure angiographique et d'établissement d'une angiographie d'une zone du corps d'un patient dans une installation de résonnance magnétique, dans lequel la zone du corps est plus grande qu'un champ de vue maximum de l'installation de résonnance magnétique et l'installation de résonnance magnétique comporte une unité de commande pour la commande de l'exécution de la mesure angiographique et de l'établissement de l'angiographie, comprenant les stades suivants :
- détection d'une grandeur anatomique du patient et d'une limite supérieure et d'une limite inférieure de la zone du corps,
- répartition de la mesure angiographique en des mesures partielles d'angiographie, dans lequel l'unité de commande répartit la zone du corps en plusieurs zones de mesure partielles à mesurer l'une après l'autre,
dans lequel chaque zone de mesure partielle est affectée à des régions du corps différentes,
dans lequel chaque zone de mesure partielle n'est pas plus grande que le champ de vue maximum et est affectée respectivement à une mesure partielle d'angiographie, et dans lequel, à chaque mesure partielle d'angiographie, sont affectés des instants de mesure comprenant respectivement un instant de début de la mesure et un instant de fin de la mesure,
de sorte qu'il est fixé une durée totale de mesure de la mesure angiographique par l'instant de début de mesure de la première mesure partielle d'angiographie et par l'instant de fin de mesure de la dernière mesure partielle d'angiographie,
- affichage des instants de début de mesure, de la durée de mesure et des instants de fin de mesure des mesures partielles d'angiographie sur une interface graphique d'utilisateur,
- modification des instants de mesure, dans lequel, lors de la modification d'un instant de mesure d'une mesure partielle d'angiographie, il s'effectue automatiquement une adaptation d'au moins un autre instant de mesure d'une autre mesure partielle d'angiographie,
- détermination automatique de paramètres de séquence des mesures partielles d'angiographie reposant sur les instants modifiés de début de mesure et/ou de fin de mesure, de manière à pouvoir effectuer la mesure partielle d'angiographie entre un instant de début de mesure et un instant de fin de mesure propres, de sorte que la mesure partielle d'angiographie doit être terminée dans la durée de mesure donnée à l'avance,
- mise à disposition des paramètres de séquence de l'unité de commande de l'installation de résonnance magnétique,
- exécution des mesures partielles d'angiographie, et
- établissement de l'angiographie à l'aide des mesures partielles d'angiographie exécutées.

2. Procédé suivant la revendication 1, dans lequel, par la modification de l'instant de fin de mesure de la mesure d'angiographie, qui n'est pas la dernière, on modifie automatiquement l'instant de début de mesure de la mesure partielle d'angiographie suivante.

3. Procédé suivant l'une des revendications précédentes, dans lequel, par la modification de l'instant de fin de mesure de la dernière mesure partielle d'angiographie, on modifie les instants de fin de mesure de toutes les mesures partielles d'angiographie et les instants de début de mesure de toutes les mesures partielles d'angiographie à l'exception de la première.

4. Procédé suivant la revendication 3, dans lequel une pause de temps de mesure, **caractérisée par** la distance entre l'instant de fin de mesure de la mesure partielle d'angiographie précédente et l'instant de début de la mesure de la mesure partielle d'angiographie suivante, reste constante et, en particulier on modifie la durée de temps de mesure de toutes les mesures partielles d'angiographie par rapport à leurs proportions à la durée d'ensemble de la mesure.

5. Procédé suivant l'une des revendications précédentes, dans lequel, par la modification de l'instant de début de mesure de la mesure partielle d'angiographie, qui n'est pas la première, on modifie automatiquement l'instant de fin de mesure de la mesure partielle d'angiographie précédente.

6. Procédé suivant l'une des revendications précédentes, dans lequel, par la modification de l'instant de début de mesure de la première mesure partielle d'angiographie, on modifie automatiquement les instants de début de mesure et les instants de fin de mesure de toutes les mesures partielles d'angiographie, en particulier sans modification de la durée d'ensemble de la mesure et des durées relatives de temps de mesure les unes par rapport aux autres.

7. Procédé suivant l'une des revendications précédentes, dans lequel, par la modification simultanée de l'instant de début de mesure et de l'instant de fin de mesure d'une mesure partielle d'angiographie ou de l'instant de fin de mesure d'une mesure partielle d'angiographie précédente et de l'instant de début de mesure d'une mesure partielle d'angiographie suivante, il s'effectue une modification de l'instant de début de mesure et de l'instant de fin de mesure de toutes les mesures partielles d'angiographie.

8. Procédé suivant l'une des revendications précédentes, dans lequel les instants de mesure des mesures partielles d'angiographie comprennent supplémentairement respectivement un instant médian, qui correspond à l'instant de la détection d'un centre d'un espace k, dans lequel on affiche l'instant médian sur l'interface graphique d'utilisateur.

9. Procédé suivant la revendication 8, dans lequel on modifie l'instant médian à l'intérieur de l'instant de début de mesure et de l'instant de fin de mesure des mesures partielles d'angiographie.

10. Procédé suivant l'une des revendications précédentes, dans lequel on détecte la limite supérieure et la limite inférieure de la zone du corps automatiquement au moyen d'une détection de repères.

11. Procédé suivant l'une des revendications précédentes, dans lequel la zone du corps comprend au moins les régions du corps abdomen, cuisse et jambe et est répartie en au moins trois zones partielles de mesure, une première zone partielle de mesure abdomen, une deuxième zone partielle de mesure cuisse et une troisième zone partielle de mesure jambe.

12. Procédé suivant l'une des revendications précédentes, dans lequel on détecte une dynamique d'agent de contraste en au moins une station de la zone du corps, en particulier en deux stations de la zone du corps, au moyen d'une mesure de bolus test avec un bolus test injecté avant la répartition de la mesure angiographique en des mesures partielles d'angiographie.

13. Procédé suivant la revendication 12, dans lequel on détecte une dynamique d'agent de contraste en deux stations de la zone du corps, et dans lequel au moins une station comprend les pieds ou les jambes du patient.

14. Procédé suivant la revendication 12 ou 13, dans lequel on représente, de manière superposée sur l'interface graphique d'utilisateur, la dynamique de l'agent de contraste et les instants de début de mesure, les durées de mesure et les instants de fin de mesure des mesures partielles d'angiographie, et/ou dans lequel on utilise la dynamique de l'agent de contraste, lors de la répartition de la mesure angiographique en des mesures partielles d'angiographie, en associant une durée de mesure plus petite à une mesure partielle d'angiographie ayant une première vitesse de l'agent de contraste déduite de la dynamique de l'agent de contraste à une mesure partielle d'angiographie ayant une deuxième vitesse de l'agent de contraste plus lente déduite de la dynamique de l'agent de contraste.

15. Installation de résonnance magnétique, constituée pour l'exécution du procédé suivant l'une des revendications précédentes, comportant :
- une unité de planification, qui est constituée :
- pour détecter une grandeur anatomique du patient et une limite supérieure et une limite inférieure de la zone du corps,
- pour la répartition de la mesure angiographique en mesures partielles d'angiographie, grâce à quoi la zone du corps est répartie en plusieurs zones partielles à mesurer les unes après les autres,
dans lequel chaque zone partielle de mesure est affectée à des régions du corps différentes,
dans lequel, à chaque mesure partielle d'angiographie, sont affectés des instants de mesure comprenant respectivement un instant de début de la mesure et un instant de fin de la mesure,
de sorte qu'il est fixé une durée totale de mesure de la mesure angiographique par l'instant de début de mesure de la première mesure partielle d'angiographie et par l'instant de fin de mesure de la dernière mesure partielle d'angiographie,
- pour la modification des instants de mesure, dans laquelle, lors de la modification d'un instant de mesure d'une mesure partielle d'angiographie, il s'effectue automatiquement une adaptation d'au moins un autre instant de mesure d'une autre mesure partielle d'angiographie,
- pour la détermination automatique des paramètres de séquence des mesures partielles d'angiographie sur la base des instants de début de mesure et/ou des instants de fin de mesure modifiés, de manière à pouvoir effectuer la mesure partielle d'angiographie entre l'instant de début de mesure et l'instant de fin de mesure, qui lui sont propres, de sorte que la mesure partielle d'angiographie doit être terminée dans la durée de mesure donnée à l'avance,
- pour la mise à disposition des paramètres de séquence d'une unité de commande,
- pour l'affichage des instants de début de mesure, de la durée de mesure et des instants de fin de mesure des mesures partielles d'angiographie sur une interface graphique d'utilisateur, et
- l'unité de commande pour la commande de l'installation de résonnance magnétique et une unité de mesure reposant sur les paramètres de séquence mis à disposition et
- l'unité de mesure pour l'exécution des mesures partielles d'angiographie et pour l'établissement de l'angiographie à l'aide des mesures partielles d'angiographie exécutées.
